# EUROPEAN PATENT APPLICATION

(11) **EP 4 759 305 A1**
(43) Date of publication of application: **17.06.2026**
(21) Application number: 24851295.6
(22) Date of filing: 31.01.2024
(51) Int. Cl.: A61K 9/16, A23L 5/00, A61K 8/73, A61K 9/20, A61K 31/4178, A61K 31/4709, A61K 31/5383, A61K 45/00, A61K 47/02, A61K 47/04, A61K 47/26, A61K 47/32, A61K 47/36, A61K 47/38, A61P 7/02, A61P 9/12, A61P 31/04, A61Q 19/00

(54) **PARTICLES AND USE THEREOF**

(30) Priority: 10.08.2023 JP 2023131381
(71) Applicant: TOWA PHARMACEUTICAL CO., LTD., Kadoma-shi, Osaka 571-8580 (JP)
(72) Inventor: YOKOYAMA, Ryoma, Kadoma-shi, Osaka 571-8580 (JP); YUMINOKI, Kayo, Kadoma-shi, Osaka 571-8580 (JP); TODA, Shogo, Kadoma-shi, Osaka 571-8580 (JP); SAEKI, Isamu, Kadoma-shi, Osaka 571-8580 (JP); OKUDA, Yutaka, Kadoma-shi, Osaka 571-8580 (JP)
(74) Representative: Graf von Stosch Patentanwaltsgesellschaft mbH
(86) International application number: PCT/JP2024/003009
(87) International publication number: WO 2025/032860

(57) **Abstract**

Provided is a particle in which a core particle is coated with a coating layer and strength of which is ensured. Further provided are an orally disintegrating tablet and the like having improved physical properties (particularly, compactibility, disintegrability, tableting failures, tablet hardness after humidification, and friability after humidification). In a particle according to the present disclosure, a core particle is coated with a coating layer and the coating layer contains cellulose nanofibers. An orally disintegrating tablet according to the present disclosure includes particles containing cellulose nanofibers having an average particle diameter of less than 10 pm.

## Description

### Technical Field

### (Invention 1)

The present invention relates to a particle having a coating layer that contains cellulose nanofibers. The present invention also relates to a composition including the particle and a method for producing the particle. Furthermore, the present invention relates to a method for ensuring strength of a particle in which a core particle is coated with a coating layer.

### (Invention 2)

The present invention relates to an orally disintegrating tablet having particles that contain cellulose nanofibers, and a disintegrative particle. The present invention also relates to a method for producing the orally disintegrating tablet and a method for improving physical properties of the orally disintegrating tablet.

### Background Art

### (Invention 1)

Core particles have problems, for example, in that bitterness may occur depending on a type of active ingredient included in the core particles and in that the core particles may not be sufficiently stable by themselves. In light of the above, a functional coating technology for coating core particles have been developed for the purposes of mitigating the bitterness caused by an active ingredient and improving stability of the core particles.

For example, Patent Literature 1 discloses a medicinal particle that is formed of a spherical core part which includes a medicinal ingredient and a coating part which coats the spherical core part and which includes a release control layer and an outermost layer containing mannitol.

### (Invention 2)

Tablets may be difficult to take for elderly and pediatric patients who have difficulty in swallowing and for patients who are on fluid restriction. Orally disintegrating tablets (also referred to as "orally disintegrating preparation: OD tablets") are applicable to such patients and are therefore useful for formulation of various active ingredients.

Orally disintegrating tablets have different properties from general tablets, and technical development of orally disintegrating tablets is carried out from perspectives (for example, compactibility, disintegrability in an oral cavity, etc.) that are different from those for general tablets. For example, Patent Literature 2 discloses, as a disintegrative particle composition that is used for orally disintegrating tablets, a disintegrative particle composition that includes a disintegrator component and micro-fibrillated cellulose.

### Citation List

### [Patent Literature]

[Patent Literature 1]
   Japanese Patent Application Publication Tokukai No. 2022-024336
[Patent Literature 2]
   International Publication No. WO 2015/163135

### Summary of Invention

### Technical Problem

### (Invention 1)

However, medicinal particles disclosed in Patent Literature 1 had room for improvement in terms of particle strength.

In light of the above, an object of an aspect of the present invention is to provide a particle in which a core particle is coated with a coating layer and which has ensured strength. Further, another object of an aspect of the present invention is to provide a method for ensuring the strength of a particle in which a core particle is coated with a coating layer.

### (Invention 2)

However, the orally disintegrating tablet disclosed in document 1 had room for improvement in physical properties of the orally disintegrating tablet, in particular, compactibility, disintegrability, tableting defects, tablet hardness after humidification, and friability after humidification.

In light of the above, a purpose of an aspect of the present invention is to provide an orally disintegrating tablet whose physical properties described above are improved, and a disintegrative particle that is used in, for example, the orally disintegrating tablet. Further, another purpose of an aspect of the present invention is to provide a method for producing an orally disintegrating tablet whose physical properties described above are improved, and a method for improving the above physical properties of an orally disintegrating tablet.

### Solution to Problem

### (Invention 1)

In order to solve the above problems, the present inventors have made diligent studies. As a result, the inventors have first found that the strength of the particle can be ensured by coating the core particle by a coating layer that contains cellulose nanofibers, and thus have accomplished an aspect of the present invention. That is, an aspect of the present invention encompasses the following configurations.
<1> A particle, wherein: a core particle is coated with a coating layer; and
   the coating layer contains cellulose nanofibers.
<2> The particle according to <1>, further including an intermediate layer between the core particle and the coating layer.
<3> The particle according to <1> or <2>, wherein the coating layer contains no additive other than the cellulose nanofibers.
<4> The particle according to any one of <1> to <3>, wherein the cellulose nanofibers have an average fiber diameter of less than 1 µm.
<5> A composition including particles recited in any one of <1> to <4>.
<6> A composition according to <5>, wherein the composition is applied to at least one selected from the group consisting of pharmaceuticals, foods, and cosmetics.
<7> A method for producing a particle in which a core particle is coated with a coating layer, the method including the step of
   coating the core particle with a coating layer containing cellulose nanofibers.
<8> A method for ensuring strength of a particle in which a core particle is coated with a coating layer, the method including the step of
   causing the coating layer to contain cellulose nanofibers.

### (Invention 2)

In order to solve the above problems, the present inventors have made diligent studies. As a result, the inventors have first found that the physical properties (particularly, compactibility, disintegrability, tableting defects, tablet hardness after humidification, and friability after humidification) of the orally disintegrating tablet are improved by containing cellulose nanofibers (hereinafter, also referred to as "CNF") having an average particle diameter of less than 10 µm, and thus have accomplished an aspect of the present invention. That is, an aspect of the present invention encompasses the following configurations.
<9> An orally disintegrating tablet including particles that contain cellulose nanofibers having an average particle diameter of less than 10 µm.
<10> The orally disintegrating tablet according to <9>, wherein the particles are disintegrative particles.
<11> The orally disintegrating tablet according to <9> or <10>, wherein the cellulose nanofibers have an average fiber diameter of 0.001 µm to 1 µm.
<12> The orally disintegrating tablet according to <9> or <10>, wherein: the particles have a core and a coating layer; and
   the cellulose nanofibers are contained in the coating layer.
<13> The orally disintegrating tablet according to <10>, wherein: the disintegrative particles include a sugar alcohol, and organic and inorganic, hydrophilic and water-insoluble additives;
   the organic hydrophilic and water-insoluble additive includes at least one selected from the group consisting of starch, a starch derivative, and crospovidone; and
   the inorganic hydrophilic and water-insoluble additive includes light anhydrous silicic acid, magnesium aluminometasilicate, or a combination thereof.
<14> An orally disintegrating tablet including cellulose nanofibers having an average fiber length of less than 10 µm.
<15> A disintegrative particle including cellulose nanofibers having an average particle diameter of less than 10 µm.
<16> A particle including a drug and cellulose nanofibers having an average particle diameter of less than 10 µm.
<17> A method for producing an orally disintegrating tablet including particles, the method including the step of
   causing the particles to contain cellulose nanofibers having an average particle diameter of less than 10 µm.
<18> A method for improving physical properties of an orally disintegrating tablet including particles, the method including the step of
   causing the particles to contain cellulose nanofibers having an average particle diameter of less than 10 µm,
   the physical properties being at least one selected from the group consisting of the following physical properties:
      (1) compactibility;
      (2) disintegrability;
      (3) tableting defects;
      (4) tablet hardness after humidification; and
      (5) friability after humidification.

### Advantageous Effects of Invention

### (Invention 1)

According to an aspect of the present invention, it is possible to provide a particle in which a core particle is coated with a coating layer and which has ensured strength. Further, an aspect of the present invention can provide a method for ensuring the strength of a particle in which a core particle is coated with a coating layer.

### (Invention 2)

According to an aspect of the present invention, it is possible to provide an orally disintegrating tablet whose various physical properties (particularly, compactibility, disintegrability, tableting defects, tablet hardness after humidification, and friability after humidification) are improved, and a disintegrative particle that is used in, for example, the orally disintegrating tablet. Further, an aspect of the present invention can provide a method for producing an orally disintegrating tablet whose physical properties described above are improved, and a method for improving the above physical properties of an orally disintegrating tablet.

### Brief Description of Drawings

Fig. 1 is a schematic view illustrating a particle according to an embodiment of the present invention in (invention 1).
Fig. 2 is a diagram showing results of dissolution tests with use of particles and tablets in Examples 1 to 3 and Comparative Examples 1 to 3.
Fig. 3 is a diagram showing results of measurement of particle strength with use of particles in Example 4 and Comparative Examples 4 to 7.
Fig. 4 is a diagram showing results of measurement of particle strength with use of particles in Example 5 and Comparative Example 8.
Fig. 5 is a diagram showing results of dissolution tests with use of particles and tablets in Example 6 and Comparative Example 9.
Fig. 6 is a diagram showing results of measurement of particle strength with use of the particles in Example 6 and Comparative Example 9.
Fig. 7 is a diagram showing results of dissolution tests with use of particles and tablets in Example 7 and Comparative Example 10.
Fig. 8 is a diagram showing results of measurement of particle strength with use of the particles in Example 7 and Comparative Example 10.
Fig. 9 is a diagram showing results of dissolution tests with use of particles and tablets in Example 8 and Comparative Example 11.
Fig. 10 is a diagram showing results of measurement of particle strength with use of the particles in Example 8 and Comparative Example 11.
Fig. 11 is a diagram obtained by capturing images of CNF (CNF-1) used in Examples of (Invention 2) with use of a scanning electron microscope (JSM-IT200, two upper images) and with use of a scanning probe microscope (SPM-Nanoa, bottom image).
Fig. 12 is a diagram showing a relationship between tablet compression force and tablet hardness (compactibility) and a relationship between tablet hardness and disintegration time (disintegrability) in Examples 1-1 to 3-1 and Comparative Examples 1-1 to 2-1.
Fig. 13 is a diagram showing tablet hardness after humidification and friability after humidification in Examples 1-1 to 3-1 and Comparative Examples 1-1 to 2-1.
Fig. 14 is a diagram showing a relationship between tablet hardness and disintegration time (disintegrability) in Examples 1-1, 4-1, and 5-1 and Comparative Examples 1-1 and 3-1.
Fig. 15 is a diagram showing tablet hardness after humidification and friability after humidification in Examples 1-1, 4-1, and 5-1 and Comparative Examples 1-1 and 3-1.
Fig. 16 is a diagram showing a relationship between tablet compression force and tablet hardness (compactibility) and a relationship between the tablet hardness and disintegration time (disintegrability) in Examples 6-1 to 7-1 and Comparative Example 4-1.
Fig. 17 is a diagram showing a relationship between tablet hardness and disintegration time (disintegrability) in Example 8-1 and Comparative Example 5-1.
Fig. 18 is a diagram showing results of measurement of particle strength with use of particles in Example 9-1 and Comparative Example 6-1.

### Description of Embodiments

The following description will discuss an embodiment of the present invention in detail. Note that any numerical range expressed as "A to B" in the present specification means "not less than A and not more than B" unless otherwise stated.

### (Invention 1)

### [1. Overview of the present invention]

A particle according to an embodiment of the present invention (hereinafter referred to as "the present particle") is characterized in that a core particle is coated with a coating layer and the coating layer contains cellulose nanofibers.

Further, a composition according to an embodiment of the present invention (hereinafter referred to as "the present composition") is characterized by including the present particles.

Furthermore, a method for producing particles in which core particles are coated with a coating layer according to an embodiment of the present invention (hereinafter referred to as "the present production method") is characterized by including the step of coating the core particles with a coating layer containing cellulose nanofibers.

In addition, a method for ensuring strength of particles in which core particles are coated with a coating layer according to an embodiment of the present invention (hereinafter referred to as "the present method") is characterized in that the coating layer contains cellulose nanofibers.

Conventionally, particles having undergone functional coating as disclosed in Patent Literature 1 had problems of film cracking in a functional coating layer due to stress caused by tableting and of consequent leakage of an active ingredient (for example, drug) that is included in a core particle. In addition, conventional particles in which core particles are coated with a coating layer also had room for improvement in terms of strength of the particles.

In light of the above, the present inventors have made diligent studies from the perspective of ensuring the strength of a particle in which a core particle is coated with a coating layer. As a result, the present inventors have successfully obtained the following findings.
- The strength of a particle can be improved by coating a core particle with a coating layer that contains cellulose nanofibers.
- In a particle including a core particle, an intermediate layer, and a coating layer, by containing cellulose nanofibers in the coating layer, it is possible to improve the strength of the particle and to prevent cracking of the intermediate layer during tableting.

It has not been known so far that cellulose nanofibers contribute to the strength of particles. Therefore, it is surprising that the present inventors focused on cellulose nanofibers for the purpose of ensuring the strength of particles, and by applying the cellulose nanofibers to the coating layer in the particles, improvement of the strength of the particles and prevention of cracking of the intermediate layer during tableting could be achieved. Further, in the case of the present particles, the strength of particles can be ensured by containing cellulose nanofibers in an outer layer (e.g., the coating layer) of core particles without adding cellulose nanofibers to the core particles.

In this way, the present particles achieves an advantageous effect on the basis of the above findings, and thus can be used in a very advantageous manner in various fields in which particles including a coating layer are used as a material.

### [2. Particle]

In the present particle, a core particle is coated with a coating layer and the coating layer contains cellulose nanofibers. The present particle can achieve an effect of ensuring the strength of the particle by containing cellulose nanofibers in the coating layer

In the present specification, "ensuring strength of a particle" means "improving strength of a particle" and/or "preventing an intermediate layer from cracking during tableting". In other words, "ensuring strength of a particle" means increasing the strength of the particle or maintaining the strength of the particle.

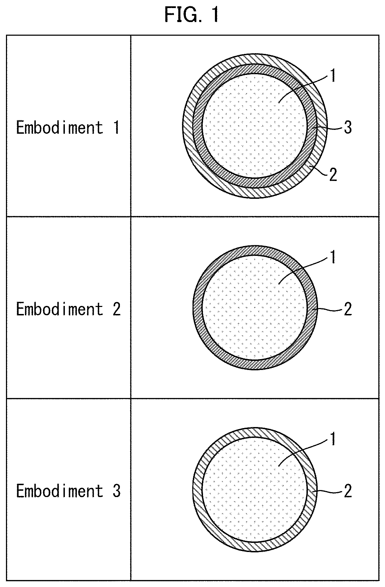

The following description will discuss embodiments of the present invention with reference to Fig. 1. Note that the following embodiments are merely examples of the present invention and the present invention is not limited to the embodiments.

Further the particle can be used in various applications including pharmaceuticals, foods, and cosmetics. The following description will discuss, as an example, the present particle which is used for pharmaceutical applications. Note that it is needless to say that the application of the present particle is not limited to pharmaceuticals.

### <Embodiment 1>

Embodiment 1 is illustrated in a top drawing of Fig. 1. In Embodiment 1, a core particle 1 is covered by an intermediate layer 3, and the intermediate layer 3 is further coated with a coating layer 2. The coating layer 2 contains cellulose nanofibers. In Embodiment 1, since the core particle 1 is coated with an intermediate layer 3 and the coating layer 2, an effect of improving particle strength is achieved. Further, an effect of making it possible to prevent the intermediate layer from cracking during tableting is achieved.

The core particle 1 is located in an innermost part of the particle and contains an active ingredient. The active ingredient contained in the core particle 1 is not particularly limited. Examples of the active ingredient include drugs, food components, nutrients, micronutrients, flavoring agents, and fragrances.

The coating layer 2 is located outside the intermediate layer 3. In an embodiment in which the particle does not include the intermediate layer 3, the coating layer 2 is located outside the core particle 1.

The coating layer 2 contains cellulose nanofibers. In the present specification, "cellulose nanofibers" mean cellulose fibers that have nanometer-sized fiber diameters and that are prepared by mechanically processing cellulose.

The cellulose nanofibers have an average fiber diameter of preferably less than 1 µm, more preferably 500 nm or less, still more preferably 300 nm or less, particularly preferably 200 nm or less, and most preferably 100 nm or less. By setting the average fiber diameter of the cellulose nanofibers to less than 1 µm, coating can be appropriately performed. In addition, the particle after the coating can maintain an appropriate size. Note that the average fiber diameter of the cellulose nanofibers means an average value of fiber diameters that are obtained by measuring any 10 or more cellulose nanofibers in an image which has been obtained by a scanning probe microscope.

The fiber length of the cellulose nanofibers is not particularly limited, and is, for example, 0.001 µm to 100 µm, preferably 0.01 µm to 50 µm, more preferably 0.05 µm to 25 µm, and still more preferably 0.1 µm to 10 µm. By setting the fiber length of the cellulose nanofibers to 0.001 µm to 100 µm, coating can be appropriately performed. In addition, the particle after the coating can maintain an appropriate size. Note that the fiber length of the cellulose nanofibers is measured by image analysis.

The content of the cellulose nanofibers is, for example, 0.1% by mass to 10.0% by mass, preferably 0.4% by mass to 9.0% by mass, more preferably 0.6% by mass to 8.0% by mass, and still more preferably 0.8% by mass to 7.0% by mass, relative to the total mass of the core particle 1 and the intermediate layer 3. In a case where the content of the cellulose nanofibers is 0.1% by mass to 10.0% by mass relative to the total mass of the core particle 1 and the intermediate layer 3, the effect of the present invention (that is, ensuring the particle strength) can be achieved.

In an embodiment of the present invention, the coating layer 2 can be a functional coating layer. The functional coating layer is not particularly limited and can be any layer that exhibits a desired function by coating the core particle 1. Examples of the coating layer include layers that contain a bitterness masking agent, an enteric base, a sustained-release base, a moisture-proof base, a light-resistant base, and a barrier base.

In an embodiment of the present invention, the coating layer 2 does not contain any additives other than the cellulose nanofibers. In other words, in an embodiment of the present invention, the coating layer 2 may be composed only of cellulose nanofibers.

The intermediate layer 3 is located between the core particle 1 and the coating layer 2. In an embodiment of the present invention, the intermediate layer 3 can be a functional coating layer. As the functional coating layer, those described in (Coating layer) is used.

The intermediate layer 3 may be a single layer or a multilayer. In a case where the intermediate layer 3 is a multilayer, the number of layers is not particularly limited. The number of layers may be, for example, two, three, four, five, or the like. In a case where the intermediate layer 3 is a multilayer, layers may be made of the same functional coating layer or different functional coating layers. Preferably, the layers are different functional coating layers.

The core particle 1, the coating layer 2, and the intermediate layer 3 in the present particle may contain an additive(s) such as an excipient, a binder, a lubricant, a disintegrator, a surfactant, a plasticizer, and/or a colorant.

The excipient is not particularly limited. Examples of the excipient include D-mannitol, lactose (for example, lactose hydrate), sucrose, corn starch, calcium phosphate, sorbitol, crystalline cellulose, and light anhydrous silicic acid. Preferably, crystalline cellulose and/or lactose (for example, lactose hydrate) are used.

The binder is not particularly limited. Examples of the binder include hydroxypropyl cellulose, hydroxypropyl methylcellulose (also referred to as "hypromellose"), povidone, methylcellulose, hydroxyethyl cellulose, carboxymethyl cellulose, polyvinylpyrrolidone, a copolymer of N-vinylpyrrolidone and vinyl acetate, and a combination of these polymers, pregelatinized starch, gelatin, agar, and gum arabic. Preferably, hypromellose is used.

The lubricant is not particularly limited. Examples of the lubricant include inert substances such as talc, kaolin, and titanium dioxide, magnesium stearate, calcium stearate, stearic acid, light anhydrous silicic acid, finely milled silicon dioxide, sodium stearyl fumarate, and glycerol fatty acid esters. Preferably, glycerin fatty acid esters are used.

The disintegrator is not particularly limited. Examples of the disintegrator include crospovidone, low-substituted hydroxypropyl cellulose, sodium starch glycolate, croscarmellose sodium, carmellose, carmellose calcium, and potato starch. Preferably, crospovidone is used.

The colorant is not particularly limited. Examples of the colorant include yellow colorants that exhibit a yellow color (for example, yellow iron sesquioxide, yellow iron oxide, Food Yellow No. 4 Aluminum Lake, and red iron oxide), red colorants that exhibit a red color (for example, iron sesquioxide, Food Red No. 2, Food Red No. 3, and Food Red No. 102), black colorants that exhibit a black color (for example, black iron oxide, carbon black, and medicinal charcoal), blue colorants that exhibit a blue color (for example, Blue No. 2 Aluminum Lake), and caramel. Preferably, yellow iron sesquioxide and/or Blue No. 2 Aluminum Lake are used.

The content of each of the additives is not particularly limited and can be appropriately set by a person skilled in the art on the basis of a conventionally known technique.

In an embodiment of the present invention, the present particle is composed of the core particle 1, a barrier layer (the intermediate layer 3), and a bitterness-masking functional coating layer (the coating layer 2) that contains cellulose nanofibers.

### <Embodiment 2>

Embodiment 2 is illustrated in a middle drawing of Fig. 1. In Embodiment 2, a core particle 1 is coated with a coating layer 2. The coating layer 2 contains cellulose nanofibers. In Embodiment 2, since the core particle 1 is coated with the coating layer 2, the effect of improving the particle strength is achieved.

In Embodiment 2, the intermediate layer 3 is not provided in the particle. The content of the cellulose nanofibers in this case is, for example, 0.1% by mass to 10.0% by mass, preferably 0.4% by mass to 9.0% by mass, more preferably 0.6% by mass to 8.0% by mass, and still more preferably 0.8 mass to 7.0 mass, relative to the mass of the core particle 1. In a case where the content of the cellulose nanofibers is 0.1% by mass to 10.0% by mass relative to the mass of the core particle 1, the effect of the present invention (that is, ensuring the particle strength) can be achieved.

In Embodiment 2, the coating layer 2 only needs to contain cellulose nanofibers, and may contain any other additives. Examples of such additives include those described in <Embodiment 1>.

Further, as the "core particle" in Embodiment 2, those described in <Embodiment 1> are used.

### <Embodiment 3>

Embodiment 3 is illustrated in a bottom drawing of Fig. 1. In Embodiment 3, a core particle 1 is coated with a coating layer 2 as in Embodiment 2. The coating layer 2 is composed only of cellulose nanofibers. In Embodiment 3, since the core particle 1 is coated with the coating layer 2, the effect of improving the particle strength is achieved.

In Embodiment 3, the intermediate layer 3 is not provided in the particle. The content of the cellulose nanofibers in this case is, for example, 0.1% by mass to 10.0% by mass, preferably 0.4% by mass to 9.0% by mass, more preferably 0.6% by mass to 8.0% by mass, and still more preferably 0.8% by mass to 7.0% by mass, relative to the mass of the core particle 1. In a case where the content of the cellulose nanofibers is 0.1% by mass to 10.0% by mass relative to the mass of the core particle 1, the effect of the present invention (that is, ensuring the particle strength) can be achieved.

Further, as the "core particle" in Embodiment 3, those described in <Embodiment 1> are used.

### [3. Composition]

The present composition includes particles described in [2. Particle] (that is, the present particles). Since the present composition contains particles whose strength is ensured, an active ingredient that is contained in the core particles can be used more effectively.

The present composition can be used for various applications on the basis of the active ingredient that is contained in the core particles of the present particles. The applications of the present composition are not particularly limited. Examples of the applications include pharmaceuticals, foods (for example, functional foods and supplements), and cosmetics.

The present composition may contain, in addition to the present particles, various additives depending on the applications. In a case where the present composition is used in a pharmaceutical application, for example, the additives described in the section of <Embodiment 1> can be used.

### [4. Method for producing particles]

The present production method is a method for producing particles in which core particles are coated with a coating layer, and includes the step of coating the core particles with a coating layer containing cellulose nanofibers. By coating the core particles with a coating layer that contains cellulose nanofibers, the present production method can ensure the particle strength.

In the present production method, a method of coating core particles with a coating layer is not particularly limited, and any method known in the present technical field can be used.

In an embodiment of the present invention, the present production method includes the step of coating the core particles with an intermediate layer.

In another embodiment of the present invention, the present production method is a method for producing particles in which core particles are coated with a coating layer, and includes the steps of: coating the core particles with an intermediate layer; and coating the intermediate layer with a coating layer containing cellulose nanofibers.

In the present method, as the "core particles", the "coating layer", the "cellulose nanofibers", and the "intermediate layer, those described in the section of [2. Particle]" are used.

### [5. Method of ensuring strength of particles]

The present method is a method for ensuring strength of the particles in which core particles are coated with a coating layer, the method being characterized in containing cellulose nanofibers in the coating layer. That is, the method includes the step of causing the coating layer to contain the cellulose nanofibers. The present method can ensure the strength of the particles by causing the coating layer to contain the cellulose nanofibers.

In the present method, a method of causing the coating layer to contain cellulose nanofibers is not particularly limited, and any method known in the present technical field can be used.

In the present method, as the "core particles", the "coating layer", and the "cellulose nanofibers", those described in the section of [2. Particle]" are used.

The present invention is not limited to the embodiments, but can be altered by a skilled person in the art within the scope of the claims. The present invention also encompasses, in its technical scope, any embodiment derived by combining technical means disclosed in differing embodiments.

### (Invention 2)

### [6. Overview of the present invention]

An orally disintegrating tablet according to an embodiment of the present invention (hereinafter, referred to as "the present orally disintegrating tablet") is characterized by having particles that contain cellulose nanofibers having an average particle diameter of less than 10 µm.

Further, a disintegrative particle according to an embodiment of the present invention (hereinafter, referred to as "the present disintegrative particle") is characterized by containing cellulose nanofibers having an average particle diameter of less than 10 µm.

Moreover, a method for producing an orally disintegrating tablet including particles according to an embodiment of the present invention (hereinafter, referred to as "the present production method") is characterized by including the step of causing the particles to contain cellulose nanofibers having an average particle diameter of less than 10 µm.

In addition, a method for improving physical properties of the orally disintegrating tablet including particles according to an embodiment of the present invention (hereinafter, referred to as "the present method") is characterized by causing the particles to contain cellulose nanofibers having an average particle diameter of less than 10 µm, the physical properties being at least one of the following physical properties: (1) compactibility, (2) disintegrability, (3) tableting defects, (4) tablet hardness after humidification, and (5) friability after humidification.

As described above, the orally disintegrating tablet disclosed in document 1 had room for improvement with respect to physical properties, in particular, the compactibility, the disintegrability, the tableting defects, the tablet hardness after humidification, and the friability after humidification, of the orally disintegrating tablet.

In light of the above, the present inventors have made diligent studies and have successfully obtained the following findings.
- By causing particles constituting an orally disintegrating tablet to contain CNF having an average particle diameter of less than 10 µm, the physical properties (particularly, compactibility, disintegrability, tableting defects, tablet hardness after humidification, and friability after humidification) of the orally disintegrating tablet are improved.
- The particles that are caused to contain CNF may be disintegrative particles or drug particles, but are preferably disintegrative particles.
- In a case where the particles have a core and a coating layer, preferably, the CNF is contained in the coating layer.

It has not been known so far that CNF contributes to various physical properties (particularly, compactibility, disintegrability, tableting defects, tablet hardness after humidification, and friability after humidification) of an orally disintegrating tablet that has particles containing the CNF. Therefore, it is surprising that the present inventors focused on CNF for the purpose of improving various physical properties of the orally disintegrating tablet and could improve the various physical properties of the orally disintegrating tablet by causing the CNF to be contained in particles constituting the orally disintegrating tablet.

As described above, since the present orally disintegrating tablet achieves an advantageous effect based on the above findings, the present orally disintegrating tablet is applicable to various active ingredients in orally disintegrating applications.

Note that in the present specification, effects (compactibility, disintegrability, tableting defects, tablet hardness after humidification, and friability after humidification) in an embodiment of the present invention may be collectively referred to as "various physical properties."

### [7. Orally disintegrating tablet]

The present orally disintegrating tablet is characterized by having particles containing CNF having an average particle diameter of less than 10 µm. The effect of making it possible to improve the various physical properties of the orally disintegrating tablet can be achieved by containing, in the present orally disintegrating tablet, CNF having the specific average particle diameter.

### (CNF)

The particles constituting the present orally disintegrating tablet contains CNF. In the present specification, the "cellulose nanofibers (CNF)" mean cellulose fibers that have nanometer-sized fiber diameters and that are prepared by mechanically processing cellulose.

The average particle diameter of the CNF is less than 10 µm, preferably 8.0 µm or less, more preferably 5.0 µm or less, and still more preferably 3.0 µm or less. By setting the average particle diameter of the CNF to less than 10 µm, the various physical properties of the orally disintegrating tablet can be improved. A lower limit of the average particle diameter of the CNF is not particularly limited as long as the effect of the present invention is achieved, and is, for example, 1.0 µm or more, and preferably 2.0 µm or more. The average particle diameter of the CNF means a volume-based average particle diameter that has been measured with use of a laser diffraction particle size distribution analyzer.

The average fiber diameter of the CNF is preferably 0.001 µm to 1 µm, more preferably 0.002 µm to 0.5 µm, still more preferably 0.005 µm to 0.2 µm, particularly preferably 0.01 µm to 0.1 µm, and most preferably 0.01 µm to 0.05 µm. By setting the average fiber diameter of the CNF to 0.001 µm to 1 µm, the various physical properties of the orally disintegrating tablet can be improved. The average fiber diameter of the CNF means an average value of fiber diameters that are obtained by measuring any 10 or more cellulose nanofibers in an image which has been obtained by a scanning probe microscope.

The CNF has an average fiber length of preferably less than 10 µm, more preferably 5 µm or less, and still more preferably 2 µm or less. By setting the average fiber length of the CNF to less than 10 µm, the various physical properties of the orally disintegrating tablet can be improved. A lower limit of the average fiber length of the CNF is not particularly limited as long as the effect of the present invention is achieved, and is, for example, 0.5 µm or more, and preferably 1.0 µm or more. The average fiber length of the CNF is measured by image analysis.

The content of the CNF, for example, 0.1% by mass to 10.0% by mass, preferably 0.6% by mass to 8.0% by mass, more preferably 0.8% by mass to 5.0% by mass, and still more preferably 1.0% by mass to 4.0% by mass, relative to the mass of the particles containing the CNF (as described later, in a case where the particles include a core and a coating layer, the mass is the total mass of the core and the coating layer). In a case where the content of the CNF is 0.1% by mass to 10.0% by mass relative to the mass of the particles containing the CNF, the various physical properties of the orally disintegrating tablet can be improved.

In an embodiment of the present invention, as the CNF contained in the present orally disintegrating tablet, commercially available CNF (for example, BiNFi-s manufactured by Sugino Machine Ltd.) can be used. The CNF contained in the present orally disintegrating tablet can also be CNF that has been produced from commercially available CNF. A method for producing the CNF contained in the present orally disintegrating tablet is not particularly limited. The CNF can be produced, for example, by micronizing general CNF. For example, in an embodiment of the present invention, the CNF can be produced by subjecting commercially available CNF (for example, CEOLUS (registered trademark) (manufactured by Asahi Kasei Corp.), PH grade) to wet micronization under a high-pressure condition (for example, 150 MPa) at a processing rate of 32 L/h to 440 L/h.

### (Particles)

The particles constituting the present orally disintegrating tablet may be disintegrative particles, drug particles, or both disintegrative particles and drug particles. That is, in the present orally disintegrating tablet, CNF may be contained in the disintegrative particle, in the drug particles, or in both the disintegrative particles and the drug particles. Further, the disintegrative particles may or may not contain a drug.

The particles constituting the present orally disintegrating tablet may include, in addition to the CNF, an additive(s) such as an excipient, a binder, a lubricant, a disintegrator, a surfactant, a plasticizer, and/or a colorant.

The excipient is not particularly limited. Examples of the excipient include D-mannitol, lactose (for example, lactose hydrate), sucrose, corn starch, calcium phosphate, sorbitol, crystalline cellulose, and light anhydrous silicic acid. Preferably, D-mannitol, crystalline cellulose, and/or lactose (for example, lactose hydrate) are used.

The binder is not particularly limited. Examples of the binder include hydroxypropyl cellulose, hydroxypropyl methylcellulose (also referred to as "hypromellose"), povidone, methylcellulose, hydroxyethyl cellulose, carboxymethyl cellulose, polyvinylpyrrolidone, a copolymer of N-vinylpyrrolidone and vinyl acetate, and a combination of these polymers, pregelatinized starch, gelatin, agar, and gum arabic. Preferably, hydroxypropyl cellulose and/or hypromellose is used.

The lubricant is not particularly limited. Examples of the lubricant include inert substances such as talc, kaolin, and titanium dioxide, magnesium stearate, calcium stearate, stearic acid, light anhydrous silicic acid, finely milled silicon dioxide, sodium stearyl fumarate, and glycerol fatty acid esters. Preferably, magnesium stearate and/or a glycerol fatty acid ester is used.

The disintegrator is not particularly limited. Examples of the disintegrator include crospovidone, low-substituted hydroxypropyl cellulose, sodium starch glycolate, croscarmellose sodium, carmellose, carmellose calcium, and potato starch. Preferably, crospovidone is used.

The colorant is not particularly limited. Examples of the colorant include yellow colorants that exhibit a yellow color (for example, yellow iron sesquioxide, yellow iron oxide, Food Yellow No. 4 Aluminum Lake, and red iron oxide), red colorants that exhibit a red color (for example, iron sesquioxide, Food Red No. 2, Food Red No. 3, and Food Red No. 102), black colorants that exhibit a black color (for example, black iron oxide, carbon black, and medicinal charcoal), blue colorants that exhibit a blue color (for example, Blue No. 2 Aluminum Lake), and caramel. Preferably, iron sesquioxide, yellow iron sesquioxide, and/or Blue No. 2 Aluminum Lake is used.

The content of each of the additives is not particularly limited and can be appropriately set by a person skilled in the art on the basis of a conventionally known technique.

In an embodiment of the present invention, the disintegrative particles may contain a sugar alcohol, and organic and inorganic, hydrophilic and water-insoluble additives. As the organic hydrophilic and water-insoluble additive, it is preferable to include at least one selected from the group consisting of starch, a starch derivative, and crospovidone. Examples of the starch and the starch derivatives include corn starch, potato starch, partially pregelatinized starch, and pregelatinized starch. As the inorganic hydrophilic and water-insoluble additive, it is preferable to contain light anhydrous silicic acid and/or magnesium aluminometasilicate. Examples of the sugar alcohol include erythritol, xylitol, sorbitol, mannitol, and lactitol.

In the present embodiment, the sugar alcohol is contained in an amount of, for example, 55% by mass to 85% by mass, and preferably 65% by mass to 75% by mass, with respect to the mass of the disintegrative particles. Further, in the present embodiment, the organic hydrophilic and water-insoluble additive is contained in an amount of, for example, 10% by mass to 40% by mass, and preferably 18% by mass to 32% by mass, with respect to the mass of the disintegrative particles. Furthermore, in the present embodiment, the inorganic hydrophilic and water-insoluble additive is contained in an amount of, for example, 0.3% by mass to 55% by mass, and preferably 0.5% by mass to 3% by mass, with respect to the mass of the disintegrative particles.

In an embodiment of the present invention, the particles constituting the present orally disintegrating tablet have a core and a coating layer. In the present embodiment, the core is coated with the coating layer. That is, the core is located in an inner part of the particle, and the coating layer is located at an outer part of the particle.

In the present embodiment, the CNF may be contained in the coating layer, in the core, or in both the coating layer and the core. From the viewpoint of further improving the various physical properties of the orally disintegrating tablet, it is preferable that the CNF be contained in the coating layer.

In the present embodiment, the core and the coating layer may contain the above-described additives in addition to the CNF. In an embodiment of the present invention, the core may contain mannitol and light anhydrous silicic acid, and the coating layer may contain corn starch, crospovidone, and CNF. In an embodiment of the present invention, the core may contain mannitol, light anhydrous silicic acid, and CNF, and the coating layer may contain corn starch and crospovidone.

In an embodiment of the present invention, the coating layer may be made of a single layer or a plurality of layers. In a case where the coating layer is made of a plurality of layers, each of coating layers may have a different function. The function of the coating layer is not particularly limited. Examples of the function include bitterness masking, an enteric base, a sustained-release base, a moisture-proof base, a light-resistant base, and a barrier base.

### (Physical properties)

In the present orally disintegrating tablet, at least one of compactibility, disintegrability, tableting defects, tablet hardness after humidification, and friability after humidification is improved. In an embodiment of the present invention, in the present orally disintegrating tablet, it is preferable that two or more selected from the group consisting of compactibility, disintegrability, tableting defects, tablet hardness after humidification, and friability after humidification be improved, and it is most preferable that all of these physical properties are improved.

In the present specification, the "compactibility" refers to a property according to which powder particles or granules bond with each other when pressure is applied to the powder or the granules, and means a characteristic that allows amorphous powder or granules to be compacted into a specific shape. It is preferable that a practical hardness can be obtained at a low pressure. Specifically, the compactibility is measured by a method which will be described in Examples.

In the present specification, the "disintegrability" means how easily a tablet disintegrates. Specifically, the disintegrability is measured by a method which will be described in the Examples.

In the present specification, the "tableting defects" means defects such as sticking that occur in a tableting process. Specifically, the tableting defects are measured by the method which will be described in the Examples.

In the present specification, the "tablet hardness after humidification" means hardness after storage for 7 days under conditions of a temperature of 25°C and a relative humidity of 75%. Specifically, the tablet hardness after humidification is measured by a method which will be described in Examples.

In the present specification, the "friability after humidification" means friability after storage for 7 days under conditions of a temperature of 25°C and a relative humidity of 75%. Specifically, the friability after humidification is measured by a method which will be described in Examples.

### [8. Disintegrative particle]

The present disintegrative particle is characterized by containing CNF having an average particle diameter of less than 10 µm. The effect of making it possible to improve the various physical properties of a composition containing the disintegrative particle can be achieved by containing, in the disintegrative particle, CNF having the above specific average particle diameter.

The present disintegrative particle can be used in various applications that require disintegration in an oral cavity. Examples of applications of the present disintegrative particle include pharmaceuticals, foods, supplements, and the like.

In the present disintegrative particle, as the "particle", the "orally disintegrating tablet", and the "cellulose nanofibers (CNF)", those described in [7. Orally disintegrating tablet] are used.

### [9. Method for producing orally disintegrating tablet]

The present production method is a method for producing an orally disintegrating tablet including particles, and includes the step of causing the particles to contain CNF having an average particle diameter of less than 10 µm. The effect of improving the various physical properties of the orally disintegrating tablet can be achieved by causing the particles constituting the orally disintegrating tablet to contain CNF having an average particle diameter of less than 10 µm.

In the present production method, a method of causing the particles constituting the orally disintegrating tablet to contain CNF is not particularly limited, and can be any method known in the present technical field. In an embodiment of the present invention, the particles are caused to contain CNF by mixing the CNF and other components constituting the particles in an arbitrary mixing device.

In the present production method, as the "particle", the "orally disintegrating tablet", and the "cellulose nanofibers (CNF)", those described in [7. Orally disintegrating tablet] are used.

### [10. Method for improving physical properties]

The present method is a method for improving physical properties of an orally disintegrating tablet including particles, and is characterized by causing the particles to contain CNF having an average particle diameter of less than 10 µm. That is, the method has a step of causing the particles to contain CNF having an average particle diameter of less than 10 µm. Further, the physical properties include at least one of (1) compactibility, (2) disintegrability, (3) tableting defects, (4) tablet hardness after humidification, and (5) friability after humidification. The present method can improve at least one of the physical properties (1) to (5) by causing the particles constituting the orally disintegrating tablet to contain CNF having an average particle diameter of less than 10 µm.

In the present method, a method of causing the particles constituting the orally disintegrating tablet to contain CNF is not particularly limited, and can be any method known in the present technical field.

In the present method, with respect to the "particles", the "orally disintegrating tablet", the "cellulose nanofibers (CNF)", and the various physical properties (physical properties (1) to (5)), the descriptions in [7. Orally disintegrating tablet] apply to them.

The present invention is not limited to the embodiments, but can be altered by a skilled person in the art within the scope of the claims. The present invention also encompasses, in its technical scope, any embodiment derived by combining technical means disclosed in differing embodiments.

### Examples

The following description will discuss an embodiment of the present invention in detail with reference to Examples. Note that the present invention is not limited to these examples.

### (Invention 1)

### [Measurement and evaluation methods]

Evaluation in the Examples and Comparative Examples was performed by the following methods.

### (Dissolution test)

Average dissolution rates of particles (overcoated particles, masked particles) and tablets were measured according to the paddle method (rotation speed: 75 rpm and second fluid for dissolution test: 900 mL) which is a method of Dissolution Test in the Japanese Pharmacopoeia. For a dissolution test of the particles, in order to prevent adhesion and aggregation of the particles during the dissolution test, the particles were mixed with disintegrative particles in equal amount in advance and were fed into a test vessel. A dissolution rate was measured with use of a fiber probe type ultraviolet-visible spectrophotometer at a measurement wavelength of 288 nm.

### (Particle strength)

Particle strength was measured with use of a micro compression tester (MCT-210, manufactured by Shimadzu Corp.).

### [Example 1]

### (Preparation of drug particles)

A 50% (w/w) ethanol solution was sprayed onto a mixed powder which included levofloxacin hydrate (0.5 hydrate), hydroxypropyl cellulose (HPC-L (75 µm to 106 µm), manufactured by Nippon Soda Co., Ltd.), and low-substituted hydroxypropyl cellulose (LH-31, manufactured by Shin-Etsu Chemical Co., Ltd.), and granulation was carried out with use of a high-speed stirring mixer. Granules thus obtained were dried with use of a tumbling fluidized bed granulation device, and then classified with use of sieves having respective mesh sizes of 250 µm and 75 µm. As a result, drug particles (core particles) were prepared.

### (Preparation of masked particles)

The drug particles thus obtained were fed into a tumbling fluidized bed granulation device. After an aminoalkyl methacrylate copolymer E (EUDRAGIT E, manufactured by Evonik Industries AG) was dissolved in a 90% (w/w) ethanol solution, talc (Micro Ace P-3, manufactured by Nippon Talc Co., Ltd.) was dispersed in that solution. This solution was sprayed onto the drug particles. As a result, the drug particles were coated with EUDRAGIT E and talc. Thus masked particles (particles in which the core particles were coated with an intermediate layer) were prepared.

### (Preparation of overcoated particles)

The masked particles thus obtained were fed into a tumbling fluidized bed granulation device. A 5% (w/v) aqueous dispersion (5% viscosity: 40000 mPa·s) of cellulose nanofibers (BiNFi-s WFo-100 5%, having an average fiber diameter of approximately 10 nm to 50 nm and a fiber length of approximately 10 µm or less, manufactured by Sugino Machine Ltd.) was diluted by adding water so as to have a concentration of 1% (w/v), and then treated with use of a homogenizer at 10000 rpm for 20 minutes. By spraying this solution, the masked particles were coated with the cellulose nanofibers. As a result, overcoated particles (particles in which the core particles were coated with an intermediate layer and a coating layer) were prepared. The coating amount of the cellulose nanofibers was 2% by mass with respect to the mass of the masked particles.

### (Preparation of disintegrative particles)

Into a fluidized bed granulation device, 71 parts by mass of D-mannitol (PEARLITOL 50C, manufactured by Roquette Japan K.K.), 2 parts by mass of ethyl cellulose (ETHOCEL Standard FP 7CPS Premium, manufactured by DuPont Nutrition & Biosciences), and 1 part by mass of light anhydrous silicic acid (Adsolider-101, manufactured by Freund Corp.) were fed. Then, a liquid in which 20 parts by mass of corn starch (Corn Starch W, manufactured by Nihon Shokuhin Kako Co., Ltd.) and 6 parts by mass of crospovidone (polyplasdone INF-10, manufactured by ISP Japan Ltd.) were dispersed in 80 parts by mass of water was sprayed, and granulation and then drying and sizing were performed. As a result, disintegrative particles were obtained.

### (Tableting)

The overcoated particles were mixed with disintegrative particles and magnesium stearate (vegetable, manufactured by Taihei Chemical Industrial Co., Ltd.). Then, tableting at 7 kN using a HANDTAB was performed. Dissolution tests were performed using the masked particles and tablets.

Table 1 shows composition of each component.

**[Table 1]**

| | Step | General Name | Comparative Example 1 | Comparative Example 2 | Comparative Example 3 |
|---|---|---|---|---|---|
| | Granulation | Levofloxacin hydrate | 25.620 | 25.620 | 25.620 |
| | | Hydroxypropyl cellulose | 3.014 | 3.014 | 3.014 |
| | | Low-substituted hydroxypropyl cellulose | 1.507 | 1.507 | 1.507 |
| | | Sub-total | 30.141 | 30.141 | 30.141 |
| Particles | Masking | Aminoalkyl methacrylate copolymer E | 1.506 | 1.506 | 1.506 |
| | | Talc | 1.506 | 1.506 | 1.506 |
| | | Sub-total | 3.012 | 3.012 | 3.012 |
| | Overcoat | Hydroxypropyl cellulose | - | 0.660 | - |
| | | Ethyl cellulose | - | - | 0.660 |
| | | Cellulose nanofibers | - | - | - |
| | | Cellulose nanofibers | - | - | - |
| | | Cellulose nanofibers | - | - | |
| | Total (mg) | | 33.153 | 33.813 | 33.813 |
| Disintegrative Particles | Granulation | Mannitol | 40.044 | 39.575 | 39.575 |
| | | Corn starch | 11.280 | 11.148 | 11.148 |
| | | Crospovidone | 3.384 | 3.344 | 3.344 |
| | | Ethyl cellulose | 1.128 | 1.115 | 1.115 |
| | | Light anhydrous silicic acid | 0.564 | 0.557 | 0.557 |
| | Total (mg) | | 56.40 | 55.74 | 55.74 |
| Lubricant | Mixing and tableting | Magnesium stearate | 0.45 | 0.45 | 0.45 |
| | Total (mg) | | 90.00 | 90.00 | 90.00 |
| | Tablet diameter (mm) | | 6 | 6 | 6 |

**[Table 1] (Continued)**

| | Step | General Name | Example 1 | Example 2 | Example 3 |
|---|---|---|---|---|---|
| | Granulation | Levofloxacin hydrate | 25.620 | 25.620 | 25.620 |
| | | Hydroxypropyl cellulose | 3.014 | 3.014 | 3.014 |
| | | Low-substituted hydroxypropyl cellulose | 1.507 | 1.507 | 1.507 |
| | | Sub-total | 30.141 | 30.141 | 30.141 |
| | Masking | Aminoalkyl methacrylate copolymer E | 1.506 | 1.506 | 1.506 |
| Particles | | Talc | 1.506 | 1.506 | 1.506 |
| | | Sub-total | 3.012 | 3.012 | 3.012 |
| | Overcoat | Hydroxypropyl cellulose | - | - | - |
| | | Ethyl cellulose | - | - | - |
| | | Cellulose nanofibers | 0.660 | - | - |
| | | Cellulose nanofibers | - | 0.660 | - |
| | | Cellulose nanofibers | - | - | 0.660 |
| | Total (mg) | | 33.813 | 33.813 | 33.813 |
| Disintegrative Particles | Granulation | Mannitol | 39.575 | 39.575 | 39.575 |
| | | Corn starch | 11.148 | 11.148 | 11.148 |
| | | Crospovidone | 3.344 | 3.344 | 3.344 |
| | | Ethyl cellulose | 1.115 | 1.115 | 1.115 |
| | | Light anhydrous silicic acid | 0.557 | 0.557 | 0.557 |
| | Total (mg) | | 55.74 | 55.74 | 55.74 |
| Lubricant | Mixing and tableting | Magnesium stearate | 0.45 | 0.45 | 0.45 |
| | Total (mg) | | 90.00 | 90.00 | 90.00 |
| | Tablet diameter (mm) | | 6 | 6 | 6 |

### [Example 2]

The same method as in Example 1 was used up to preparation of masked particles.

### (Preparation of overcoated particles)

The masked particles thus obtained were fed into a tumbling fluidized bed granulation device. A 5% (w/v) aqueous dispersion (5% viscosity: 20000 mPa·s) of cellulose nanofibers (BiNFi-s AFo-100 5%, having an average fiber diameter of approximately 10 nm to 50 nm and a fiber length of approximately 0.5 µm to 1.0 µm, manufactured by Sugino Machine Ltd.) was diluted by adding water so as to have a concentration of 1% (w/v), and then treated with use of a homogenizer at 10000 rpm for 20 minutes. By spraying this solution, the masked particles were coated with the cellulose nanofibers. As a result, overcoated particles were prepared. The coating amount of the cellulose nanofibers was 2% by mass with respect to the mass of the masked particles.

Next, disintegrative particles were prepared and tableting was performed, as in Example 1. Dissolution tests were performed using the overcoated particles and tablets.

### [Example 3]

The same method as in Example 1 was used up to preparation of masked particles.

### (Preparation of overcoated particles)

The masked particles thus obtained were fed into a tumbling fluidized bed granulation device. A 2% (w/v) aqueous dispersion that had a viscosity of 1000 mPa·s or more and that contained crystalline cellulose which had been made into cellulose nanofibers was diluted by adding water so as to have a concentration of 1% (w/v), and then, treated with a homogenizer at 10000 rpm for 20 minutes. By spraying this solution, the masked particles were coated with the crystalline cellulose, which had been made into cellulose nanofibers. As a result, overcoated particles were prepared. The coating amount of the crystalline cellulose, which had been made into cellulose nanofibers, was 2% by mass with respect to the mass of the masked particles.

Next, disintegrative particles were prepared and tableting was performed, as in Example 1. Dissolution tests were performed using the overcoated particles and tablets.

### [Comparative Example 1]

The same method as in Example 1 was used up to preparation of masked particles. In this comparative example, overcoated particles were not prepared.

Next, disintegrative particles were prepared as in Example 1.

### (Tableting)

The masked particles were mixed with the disintegrative particles and magnesium stearate (vegetable, manufactured by Taihei Chemical Industrial Co., Ltd.). Then, tableting at 7 kN using a HANDTAB was performed. Dissolution tests were performed using the masked particles and tablets.

### [Comparative Example 2]

The same method as in Example 1 was used up to preparation of masked particles.

### (Preparation of overcoated particles)

Hydroxypropyl cellulose (HPC-SL, manufactured by Nippon Soda Co., Ltd.) was dissolved in water so that a concentration became 1% (w/v). This solution was sprayed to coat the masked particles with HPC-SL. As a result, overcoated particles were prepared. The coating amount of the HPC-SL was 2% by mass with respect to the mass of the masked particles.

Next, disintegrative particles were prepared and tableting was performed, as in Example 1. Dissolution tests (RT-J2000, manufactured by Dai-Nippon Seiki Co., Ltd.) were performed using the overcoated particles and tablets.

### [Comparative Example 3]

The same method as in Example 1 was used up to preparation of masked particles.

### (Preparation of overcoated particles)

Ethyl cellulose (ETHOCEL Standard FP 7CPS Premium, manufactured by DuPont Nutrition & Biosciences) was dissolved in a 90% (w/w) ethanol so that a concentration of a resultant solution became 1% (w/v). By spraying this solution, the masked particles were coated with the ethyl cellulose. As a result, overcoated particles were prepared. The coating amount of the ethyl cellulose was 2% by mass with respect to the mass of the masked particles.

Next, disintegrative particles were prepared and tableting was performed, as in Example 1. Dissolution tests were performed using the overcoated particles and tablets.

### [Result-1]

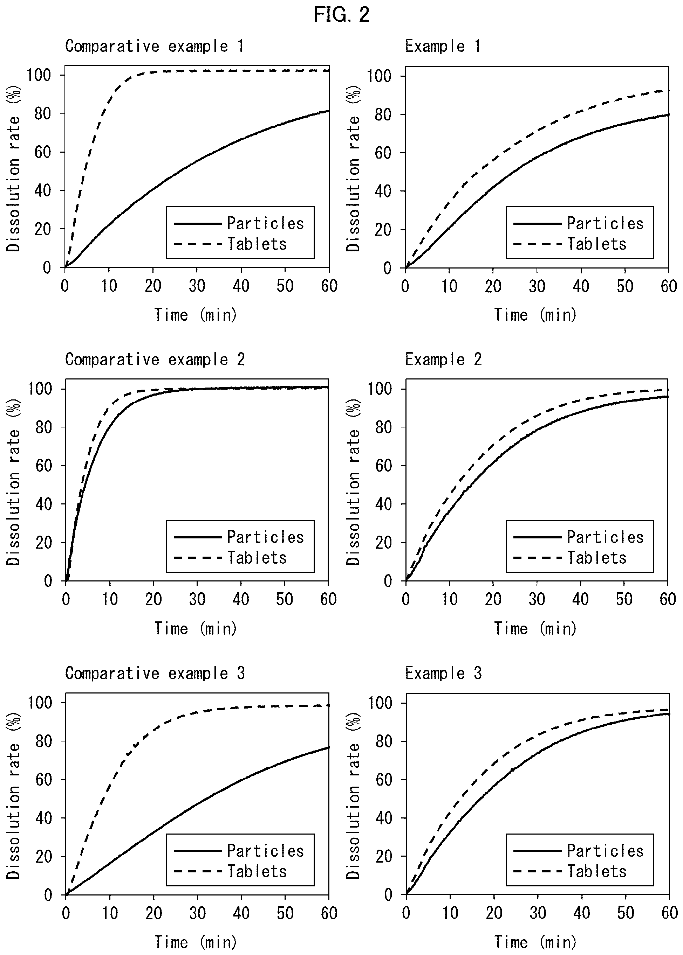

Fig. 2 shows results of dissolution tests performed for the particles (overcoated particles and masked particles) and the tablets which were produced in Examples 1 to 3 and Comparative Examples 1 to 3.

It is clear from a comparison of Examples and Comparative Examples in Fig. 2 that a difference in dissolution behavior between the particles and the tablets is smaller in Examples 1 to 3 than in Comparative Examples 1 to 3. A small difference in dissolution behavior between the particles and the tablets means that cracking of a masking layer (intermediate layer) during tableting can be prevented. Therefore, it is shown that the particles according to an embodiment of the present invention, which include a core particle, an intermediate layer, and a coating layer and in which the coating layer contains cellulose nanofibers, can prevent cracking of the intermediate layer during tableting (that is, the strength of the particles can be ensured).

### [Example 4]

Particle strength was measured, with use of overcoat particles obtained as in Example 1.

### [Comparative Example 4]

Drug particles were prepared as in Example 1. Particle strength was measured with use of the drug particles obtained.

### [Comparative Example 5]

Particle strength was measured, with use of masked particles obtained as in Comparative Example 1.

### [Comparative Example 6]

Particle strength was measured, with use of overcoat particles obtained as in Comparative Example 2.

### [Comparative Example 7]

Particle strength was measured, with use of overcoat particles obtained as in Comparative Example 3.

### [Example 5]

The same method as in Example 1 was used up to preparation of drug particles.

### (Preparation of masked particles)

The drug particles thus obtained were fed into a tumbling fluidized bed granulation device. A 5% (w/v) aqueous dispersion of cellulose nanofibers (BiNFi-s WFo-100 5%, manufactured by Sugino Machine Ltd.) was diluted by adding water so as to have a concentration of 1% (w/v), and then treated with use of a homogenizer at 10000 rpm for 20 minutes. By spraying this solution, the drug particles were coated with the cellulose nanofibers. As a result, masked particles (particles in which the core particles were coated with a coating layer) were prepared. The coating amount of the cellulose nanofibers was 2% by mass with respect to the mass of the drug particles.

Particle strength was measured, with use of the masked particles obtained.

### [Comparative Example 8]

Drug particles were prepared as in Example 1. Particle strength was measured with use of the drug particles obtained.

### [Result-2]

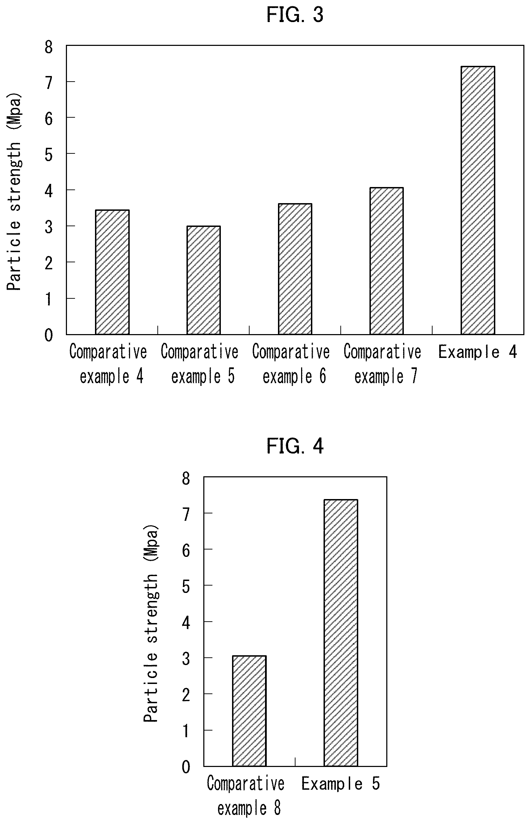

Figs. 3 and 4 show results of measuring the particle strength of the particles (drug particles, masked particles, and overcoated particles) which were produced in Examples 4 and 5 and Comparative Examples 4 to 8.

It is clear from Fig. 3 that the particle strength is higher in Example 4 than in Comparative Examples 4 to 7. Therefore, it is shown that the particles according to an embodiment of the present invention, which include a core particle, an intermediate layer, and a coating layer and in which the coating layer contains cellulose nanofibers, have an improved particle strength.

Further, it is clear from Fig. 4 that the particle strength is higher in Example 5 than in Comparative Example 8. Therefore, it is shown that the particles according to an embodiment of the present invention in which the core particles are coated with a coating layer containing cellulose nanofibers have an improved particle strength.

### [Example 6]

The same method as in Example 1 was used up to preparation of drug particles.

### (Preparation of masked particles)

The drug particles thus obtained were fed into a tumbling fluidized bed granulation device. A 5% (w/v) aqueous dispersion of cellulose nanofibers (BiNFi-s AFo-100 5%, manufactured by Sugino Machine Ltd.) was diluted 3-fold by adding ethanol, and then treated with use of a homogenizer at 10000 rpm for 20 minutes. As a result, a cellulose nanofiber-containing solution was obtained. Next, an aminoalkyl methacrylate copolymer E (EUDRAGIT E, manufactured by Evonik Industries AG) was dissolved in an ethanol solution, and the cellulose nanofiber-containing solution was added to finally make an 80% (w/w) ethanol solution. This liquid was sprayed onto the drug particles to coat the drug particles with EUDRAGIT E and cellulose nanofibers. As a result, masked particles were prepared.

Next, disintegrative particles were prepared and tableting was performed, as in Example 1. Dissolution tests were performed using the masked particles and tablets obtained. Particle strength was measured, with use of the masked particles obtained.

Table 2 shows composition of each component.

**[Table 2]**

| | Step | General Name | Comparative Example 9 | Example 6 |
|---|---|---|---|---|
| | Granulation | Levofloxacin hydrate | 25.620 | 25.620 |
| | | Hydroxypropyl cellulose | 3.014 | 3.014 |
| | | Low-substituted hydroxypropyl cellulose | 1.507 | 1.507 |
| Particles | | Sub-total | 30.141 | 30.141 |
| | Masking | Aminoalkyl methacrylate copolymer E | 3.014 | 3.014 |
| | | Talc | 0.603 | - |
| | | Cellulose nanofibers | - | 0.603 |
| | | Sub-total | 3.617 | 3.617 |
| | Total (mg) | | 33.758 | 33.758 |
| Disintegrative Particles | Granulation | Mannitol | 39.612 | 39.612 |
| | | Corn starch | 11.158 | 11.158 |
| | | Crospovidone | 3.348 | 3.348 |
| | | Ethyl cellulose | 1.116 | 1.116 |
| | | Light anhydrous silicic acid | 0.558 | 0.558 |
| | Total (mg) | | 55.79 | 55.79 |
| Lubricant | Mixing and tableting | Magnesium stearate | 0.45 | 0.45 |
| | Total (mg) | | 90.00 | 90.00 |
| | Tablet diameter (mm) | | 6 | 6 |

### [Comparative Example 9]

The same method as in Example 1 was used up to preparation of drug particles.

### (Preparation of masked particles)

The drug particles thus obtained were fed into a tumbling fluidized bed granulation device. After an aminoalkyl methacrylate copolymer E (EUDRAGIT E, manufactured by Evonik Industries AG) was dissolved in an 80% (w/w) ethanol solution, talc (Micro Ace P-3, manufactured by Nippon Talc Co., Ltd.) was dispersed in that solution. This solution was sprayed onto the drug particles. As a result, the drug particles were coated with EUDRAGIT E and talc. Thus masked particles (particles in which the core particles were coated with a coating layer) were prepared.

Next, disintegrative particles were prepared and tableting was performed, as in Example 1. Dissolution tests were performed using the masked particles and tablets obtained. Particle strength was measured, with use of the masked particles obtained.

### [Result-3]

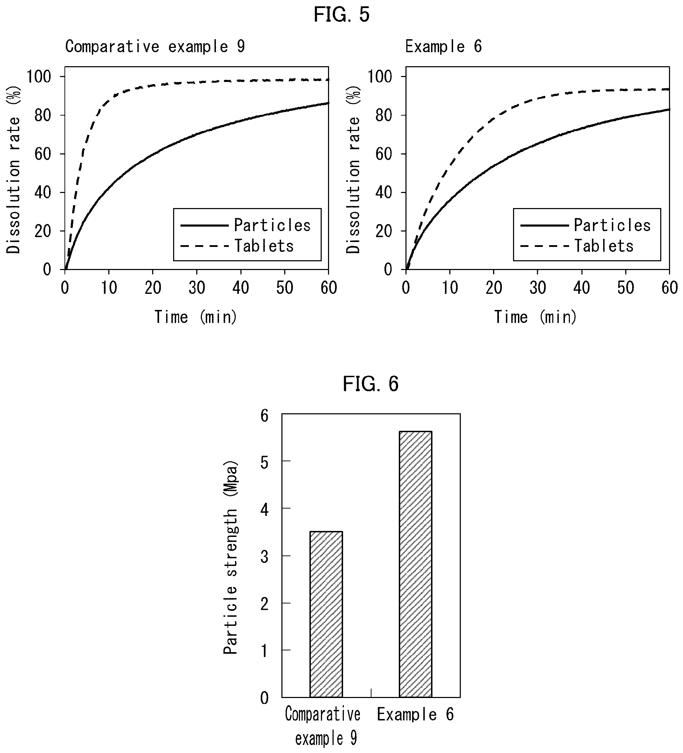

Figs. 5 and 6 show results of measuring the dissolution rate and the particle strength of the particles (masked particles) and the tablets which were produced in Example 6 and Comparative Example 9.

It is clear from Fig. 5 that a difference in dissolution behavior between the particles and the tablets is smaller in Example 6 than in Comparative Example 9. Therefore, it is shown that the particles according to an embodiment of the present invention, which include a core particle and a coating layer and in which the coating layer contains cellulose nanofibers, can prevent cracking of the coating layer during tableting.

Further, it is clear from Fig. 6 that the particle strength is higher in Example 6 than in Comparative Example 9. Therefore, it is shown that the particles according to an embodiment of the present invention, which include a core particle and a coating layer and in which the coating layer contains cellulose nanofibers, have an improved particle strength.

### [Example 7]

The same method as in Example 1 was used up to preparation of drug particles.

### (Preparation of masked particles)

The drug particles thus obtained were fed into a tumbling fluidized bed granulation device. After an aminoalkyl methacrylate copolymer RL and an aminoalkyl methacrylate copolymer RS (EUDRAGIT RL and RS, manufactured by Evonik Industries AG) were dissolved in a 90% (w/w) ethanol solution, talc (Micro Ace P-3, manufactured by Nippon Talc Co., Ltd.) was dispersed in that solution. By spraying this solution on the drug particles, masked particles (particles in which the core particles were coated with an intermediate layer) were prepared.

### (Preparation of overcoated particles)

The masked particles thus obtained were fed into a tumbling fluidized bed granulation device. A 2% (w/v) aqueous dispersion (2% viscosity: 6000 mPa·s) of cellulose nanofibers (BiNFi-s WFo-100 2%, manufactured by Sugino Machine Ltd.) was diluted by adding water so as to have a concentration of 1% (w/v), and then treated with use of a homogenizer at 10000 rpm for 20 minutes. By spraying this solution, overcoated particles (particles in which the core particles were coated with an intermediate layer and a coating layer) were prepared. The coating amount of the cellulose nanofibers was 2% by mass with respect to the mass of the masked particles.

Next, disintegrative particles were prepared and tableting (tablet compression force: 11 kN) was performed, as in Example 1. Dissolution tests were performed using the overcoated particles and tablets.

Table 3 shows composition of each component.

**[Table 3]**

| | Step | General Name | Comparative Example 10 | Example 7 |
|---|---|---|---|---|
| | Granulation | Levofloxacin hydrate | 25.620 | 25.620 |
| | | Hydroxypropyl cellulose | 3.014 | 3.014 |
| | | Low-substituted hydroxypropyl cellulose | 1.507 | 1.507 |
| | | Sub-total | 30.141 | 30.141 |
| | Masking | Aminoalkyl methacrylate copolymer RL | 6.080 | 6.080 |
| Particles | | Aminoalkyl methacrylate copolymer RS | 6.080 | 6.080 |
| | | Talc | 6.080 | 6.080 |
| | | Sub-total | 18.240 | 18.240 |
| | Overcoat | Cellulose nanofibers | - | 0.968 |
| | Total (mg) | | 48.381 | 49.349 |
| Disintegrative Particles | Granulation | Mannitol | 45.476 | 44.787 |
| | | Corn starch | 12.810 | 12.616 |
| | | Crospovidone | 3.843 | 3.785 |
| | | Ethyl cellulose | 1.281 | 1.262 |
| | | Light anhydrous silicic acid | 0.641 | 0.631 |
| | Total (mg) | | 64.05 | 63.08 |
| Lubricant | Mixing and tableting | Magnesium stearate | 0.57 | 0.57 |
| | Total (mg) | | 113.00 | 113.00 |
| | Tablet diameter (mm) | | 6.5 | 6.5 |

### [Comparative Example 10]

The same method as in Example 7 was used up to preparation of masked particles. In this comparative example, overcoated particles were not prepared.

Next, disintegrative particles were prepared and tableting (tablet compression force: 11 kN) was performed, as in Example 1. Dissolution tests were performed using the masked particles and tablets.

### [Result-4]

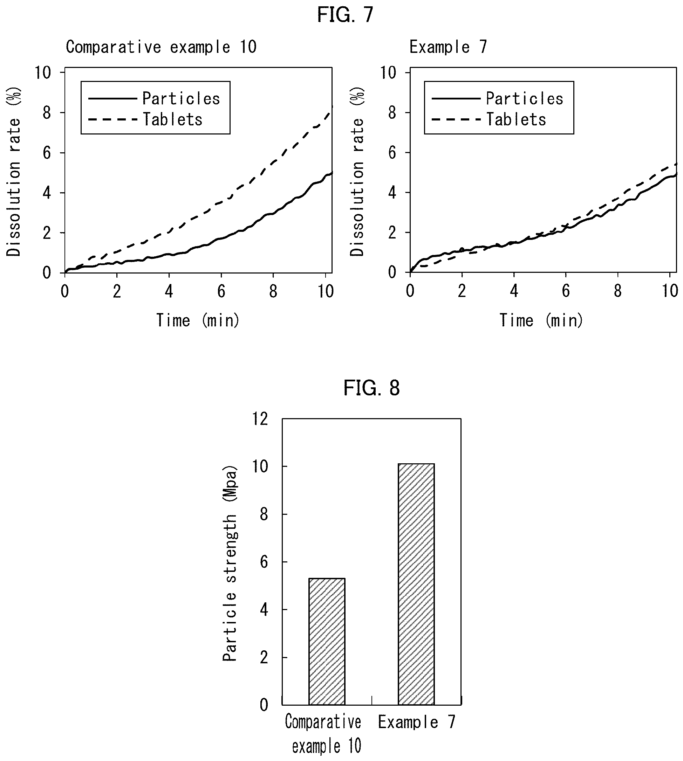

Figs. 7 and 8 show results of measuring the dissolution rate and the particle strength of the particles (overcoated particles) and the tablets which were produced in Example 7 and Comparative Example 10.

It is clear from Fig. 7 that a difference in dissolution behavior between the particles and the tablets is smaller in Example 7 than in Comparative Example 10. Therefore, it is shown that the particles according to an embodiment of the present invention, which include a core particle, an intermediate layer, and a coating layer and in which the coating layer contains cellulose nanofibers, can prevent cracking of the coating layer during tableting.

It is clear from Fig. 8 that the particle strength is higher in Example 7 than in Comparative Example 10. Therefore, it is shown that the particles according to an embodiment of the present invention, which include a core particle, an intermediate layer, and a coating layer and in which the coating layer contains cellulose nanofibers, have an improved particle strength.

### [Example 8]

The same method as in Example 1 was used up to preparation of drug particles.

### (Preparation of masked particles)

The drug particles thus obtained were fed into a tumbling fluidized bed granulation device. After ethyl cellulose (ETHOCEL Standard FP 7CPS Premium, manufactured by DuPont Nutrition & Biosciences) was dissolved in a 90% (w/w) ethanol solution, talc (Micro Ace P-3, manufactured by Nippon Talc Co., Ltd.) and silicon dioxide hydrate (Adsolider-102, manufactured by Freund Corp.) were dispersed in that solution. This solution was then sprayed onto the drug particles. As a result, masked particles (particles in which core particles were coated with a coating layer) were prepared.

### (Preparation of overcoated particles)

The masked particles thus obtained were fed into a tumbling fluidized bed granulation device. A 5% (w/v) aqueous dispersion (5% viscosity: 20000 mPa·s) of cellulose nanofibers (BiNFi-s AFo-100 5%, manufactured by Sugino Machine Ltd.) was diluted by adding water so as to have a concentration of 1% (w/v), and then treated with use of a homogenizer at 10000 rpm for 20 minutes. By spraying this solution onto the masked particles, overcoated particles (particles in which the core particles were coated with an intermediate layer and a coating layer) were prepared. The coating amount of the cellulose nanofibers was 2% by mass with respect to the mass of the masked particles.

Next, disintegrative particles were prepared and tableting (tablet compression force: 7 kN) was performed, as in Example 1. Dissolution tests were performed using the overcoated particles and tablets.

Table 4 shows composition of each component.

**[Table 4]**

| | Step | General Name | Comparative Example 11 | Example 8 |
|---|---|---|---|---|
| | Granulation | Levofloxacin hydrate | 25.620 | 25.620 |
| | | Hydroxypropyl cellulose | 3.014 | 3.014 |
| | | Low-substituted hydroxypropyl cellulose | 1.507 | 1.507 |
| | | Sub-total | 30.141 | 30.141 |
| | Masking | Ethyl cellulose | 0.964 | 0.964 |
| Particles | | Talc | 0.362 | 0.362 |
| | | Silicon dioxide hydrate | 0.181 | 0.181 |
| | | Sub-total | 1.507 | 1.507 |
| | Overcoat | Cellulose nanofibers | - | 0.633 |
| | Total (mg) | | 31.648 | 32.281 |
| Disintegrative Particles | Granulation | Mannitol | 41.109 | 40.662 |
| | | Corn starch | 11.580 | 11.454 |
| | | Crospovidone | 3.474 | 3.436 |
| | | Ethyl cellulose | 1.158 | 1.145 |
| | | Light anhydrous silicic acid | 0.579 | 0.573 |
| | Total (mg) | | 57.90 | 57.27 |
| Lubricant | Mixing and tableting | Magnesium stearate | 0.45 | 0.45 |
| | Total (mg) | | 90.00 | 90.00 |
| | Tablet diameter (mm) | | 6 | 6 |

### [Comparative Example 11]

The same method as in Example 8 was used up to preparation of masked particles. In this comparative example, overcoated particles were not prepared.

Next, disintegrative particles were prepared and tableting (tablet compression force: 7 kN) was performed, as in Example 1. Dissolution tests were performed using the overcoated particles and tablets.

### [Result-5]

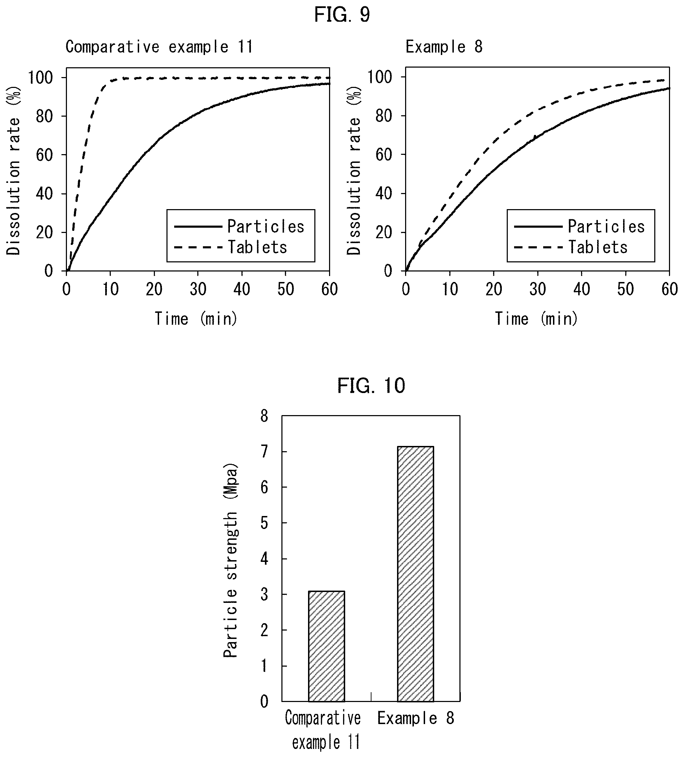

Figs. 9 and 10 show results of measuring the dissolution rate and the particle strength of the particles (overcoated particles) and the tablets which were produced in Example 8 and Comparative Example 11.

It is clear from Fig. 9 that a difference in dissolution behavior between the particles and the tablets is smaller in Example 8 than in Comparative Example 11. Therefore, it is shown that the particles according to an embodiment of the present invention, which include a core particle, an intermediate layer, and a coating layer and in which the coating layer contains cellulose nanofibers, can prevent cracking of the coating layer during tableting.

Further, it is clear from Fig. 10 that the particle strength is higher in Example 8 than in Comparative Example 11. Therefore, it is shown that the particles according to an embodiment of the present invention, which include a core particle, an intermediate layer, and a coating layer and in which the coating layer contains cellulose nanofibers, have an improved particle strength.

### (Invention 2)

### [Materials]

### (CNF)

### <CNF used in Examples>

- CNF-1: Average particle diameter was 2.0 µm, average fiber diameter was approximately 10 nm, and average fiber length was less than 10 µm. Crystalline cellulose (Ceolus (registered trademark) (manufactured by Asahi Kasei Corp.), grade PH-101, having an average particle diameter of 77 µm, an average fiber diameter of approximately 50 µm, and an average fiber length of approximately 150 µm) was dispersed in water so that the concentration of a resultant solution became 2% (w/v), and then treated 30 times with a wet pulverization and dispersion device (Star Burst Labo, manufactured by Sugino Machine Ltd.) to produce a CNF aqueous dispersion.
- CNF-2: BiNFi-s (manufactured by Sugino Machine Ltd.), grade short chain, having an average particle diameter of 2.9 µm, an average fiber diameter of approximately 10 nm to 50 nm, and an average fiber length of 0.5 µm to 1.0 µm.
- CNF-3: BiNFi-s (manufactured by Sugino Machine Ltd.), grade very short chain, having an average particle diameter of 2.5 µm, an average fiber diameter of approximately 10 nm to 50 nm, and an average fiber length of less than 0.5 µm.

### <Cellulose used in Comparative Examples>

- Microfibrillated cellulose: CELISH (manufactured by Daicel Corp.), grade FD200L, having an average particle diameter of 11.1 µm, an average fiber diameter of approximately 0.1 µm to 1.0 µm; and an average fiber length of more than 10 µm. Microfibrillated cellulose used in Examples of International Publication No. WO 2015/163135.
- Crystalline cellulose: Ceolus, grade PH-101 (manufactured by Asahi Kasei Corp.)
- Ethyl cellulose: ETHOCEL Standard 7 FP Premium (manufactured by DuPont Nutrition & Biosciences)

### [Measurement and evaluation methods]

Evaluation in the Examples and Comparative Examples was performed by the following methods.

### (Average particle diameter)

A CNF-containing aqueous dispersion was prepared to have a solid content of 0.125% to 0.5%. After preparation, treatment using an ultrasonic homogenizer (UD-200, manufactured by Tomy Seiko Co., Ltd.) was carried out for 5 minutes to 10 minutes until fibers were uniformly dispersed. Then, a treated liquid was subjected to measurement using a laser diffraction particle size distribution analyzer (MS3000, manufactured by Malvern Panalytical Ltd.) equipped with a wet dispersion unit (Hydro MV, manufactured by Malvern Panalytical Ltd.). After the liquid to be measured was circulated for 30 seconds at a pump speed of 3500 rpm while irradiation with ultrasonic was carried out, the ultrasound and the pump were stopped, and the average particle diameter was measured. After the liquid to be measured was circulated again, the measurement was performed in the same manner. As a result, the average particle diameter was obtained.

### (Average fiber diameter)

For observation of form of CNF and measurement of the fiber diameter of the CNF, a scanning probe microscope was used. As a pretreatment for an observation sample, a sheet obtained by drying a CNF aqueous dispersion was fixed to a sample holder, and a fine probe was brought into contact with a surface of the sample and caused to scan the surface. Thus, a surface fine structure (e.g., fiber diameter) was observed and measured at nanometer-scale resolution.

### (Compactibility)

Tablet hardness was measured with use of a hardness tester (manufactured by ERWEKA). As a result, the compactibility was evaluated.

### (Disintegrability)

A disintegrability test was performed without an auxiliary disk in accordance with a method of Disintegration Test (for rapidly disintegrating preparations) of the 18th revised Japanese Pharmacopoeia. Thus, a disintegration time of tablets was measured.

### (Tablet hardness after humidification)

After storage for 7 days under conditions of a temperature of 25°C and a relative humidity of 75%, the tablet hardness was measured with use of a hardness tester (manufactured by ERWEKA).

### (Friability after humidification)

After storage for 7 days under conditions of a temperature of 25°C and a relative humidity of 75%, the friability was measured with use of a friability tester (manufactured by Toyama Sangyo Co., Ltd.).

### (Tableting defects)

Continuous tableting was performed by a rotary tableting machine, and visual inspection as to tablet appearance defects and powder that has adhered to, for example, a punch and a die were carried out. Specifically, after tableting for 20 minutes, the punch of the rotary tableting machine was visually inspected, and cloudiness of the punch (adhesion of powder) was evaluated. The following were evaluation criteria.

### (Evaluation criteria)

A: No powder adhesion.
B: Powder adhered thinly, resulting in a cloudy state (a state in which metal luster of a surface of the punch is lost).
C: A state in which powder adhesion is clearly visible.

### (Particle strength)

Particle strength was measured with use of a micro compression tester (MCT-210, manufactured by Shimadzu Corp.).

### [Example 1-1]

Purified water was sprayed onto a mixed powder which included olmesartan medoxomil, hydroxypropyl cellulose (HPC-M, manufactured by Nippon Soda Co., Ltd.), and lactose hydrate (Fine Powder, manufactured by DFE Pharma K.K.). Then, a resultant mixture was granulated with use of a high-speed stirring mixer. Granules thus obtained were dried with use of a fluidized bed granulation device (MP-01, manufactured by Powrex Corp.) and then classified with use of sieves having respective mesh sizes of 250 µm and 75 µm. As a result, drug particles were prepared.

Next, 71 parts by mass of D-mannitol (PEARLITOL 50C, manufactured by Roquette Japan K.K.) and 1 part by mass of light anhydrous silicic acid (Adsolider-101, manufactured by Freund Corp.) were fed into a fluidized bed granulation device. CNF-1 was diluted by adding water so as to have a concentration of 1.25% (w/v), and then treated with a homogenizer at 10,000 rpm for 20 minutes (2 parts by mass as CNF). In this liquid, 20 parts by mass of corn starch (Corn Starch W, manufactured by Nihon Shokuhin Kako Co., Ltd.) and 6 parts by mass of crospovidone (polyplasdone INF-10, manufactured by ISP Japan Ltd.) were dispersed in water. A liquid thus obtained was sprayed, and granulation and then drying and sizing at 30 M were performed. As a result, disintegrative particles were obtained.

Thereafter, the drug particles, the disintegrative particles, aspartame (manufactured by Ajinomoto Co., Inc.), light anhydrous silicic acid (Adsolider-101, manufactured by Freund Corp.), and magnesium stearate (vegetable, manufactured by Taihei Chemical Industrial Co., Ltd.) were mixed, and then tableted by a rotary tableting machine to a practical hardness. As a result, tablets with a diameter of 10 mm (punch: with marking, with score line) were obtained. The tablets thus obtained were measured for compactibility, disintegrability, tablet hardness after humidification, friability after humidification, and tableting defects.

Table 5 shows composition of each component.

**[Table 5]**

| | Step | General Name | Example 1-1 | Example 2-1 | Example 3-1 | Example 4-1 |
|---|---|---|---|---|---|---|
| Drug particles | Granulation | Olmesartan medoxomil | 40.00 | 40.00 | 40.00 | 40.00 |
| | | Lactose hydrate | 40.00 | 40.00 | 40.00 | 40.00 |
| | | Hydroxypropyl cellulose | 1.00 | 1.00 | 1.00 | 1.00 |
| | | Total (mg) | 81.00 | 81.00 | 81.00 | 81.00 |
| | | Mannitol | 187.30 | 189.93 | 182.01 | 187.30 |
| | | Corn starch | 52.76 | 52.76 | 52.76 | 52.76 |
| | | Crospovidone | 15.83 | 15.83 | 15.83 | 15.83 |
| | | Light anhydrous silicic acid | 2.64 | 2.64 | 2.64 | 2.64 |
| Disintegrative Particles | Granulation | Cellulose nanofibers (CNF-1) | 5.28 | 2.64 | 10.56 | - |
| | | Cellulose nanofibers (CNF-2) | - | - | - | 5.28 |
| | | Cellulose nanofibers (CNF-3) | - | - | - | - |
| | | Microfibrillated cellulose | - | - | - | - |
| | | Ethyl cellulose | - | - | - | - |
| | | Crystalline cellulose | - | - | - | - |
| | | Total (mg) | 263.80 | 263.80 | 263.80 | 263.80 |
| Sweetening agent | Mixing and tableting | Aspartame | 10.80 | 10.80 | 10.80 | 10.80 |
| Fluidizing agent | | Light anhydrous silicic acid | 0.90 | 0.90 | 0.90 | 0.90 |
| Lubricant | | Magnesium stearate | 3.6 | 3.6 | 3.6 | 3.6 |
| | Total (mg) | | 360 | 360 | 360 | 360 |
| | Tablet diameter (mm) | | 10 | 10 | 10 | 10 |

**[Table 5] (continued)**

| | Step | General Name | Example 5-1 | Example 6-1 | Example 7-1 |
|---|---|---|---|---|---|
| Drug particles | Granulation | Olmesartan medoxomil | 40.00 | 40.00 | 40.00 |
| | | Lactose hydrate | 40.00 | 40.00 | 40.00 |
| | | Hydroxypropyl cellulose | 1.00 | 1.00 | 1.00 |
| | | Total (mg) | 81.00 | 81.00 | 81.00 |
| | | Mannitol | 187.30 | 187.30 | 187.30 |
| | | Corn starch | 52.76 | 52.76 | 52.76 |
| | | Crospovidone | 15.83 | 15.83 | 15.83 |
| | | Light anhydrous silicic acid | 2.64 | 2.64 | 2.64 |
| | | Cellulose nanofibers (CNF-1) | - | 5.28 | 5.28 |
| Disintegrative Particles | Granulation | Cellulose nanofibers (CNF-2) | - | - | - |
| | | Cellulose nanofibers (CNF-3) | 5.28 | - | - |
| | | Microfibrillated cellulose | | | |
| | | Ethyl cellulose | - | - | - |
| | | Crystalline cellulose | - | - | - |
| | | Total (mg) | 263.80 | 263.80 | 263.80 |
| Sweetening agent | Mixing and tableting | Aspartame | 10.80 | 10.80 | 10.80 |
| Fluidizing agent | | Light anhydrous silicic acid | 0.90 | 0.90 | 0.90 |
| Lubricant | | Magnesium stearate | 3.6 | 3.6 | 3.6 |
| | Total (mg) | | 360 | 360 | 360 |
| | Tablet diameter (mm) | | 10 | 10 | 10 |

**[Table 5] (continued)**

| | Step | General Name | Comparative Example 1-1 | Comparative Example 2-1 | Comparative Example 3-1 | Comparative Example 4-1 |
|---|---|---|---|---|---|---|
| Drug particles | Granulation | Olmesartan medoxomil | 40.00 | 40.00 | 40.00 | 40.00 |
| | | Lactose hydrate | 40.00 | 40.00 | 40.00 | 40.00 |
| | | Hydroxypropyl cellulose | 1.00 | 1.00 | 1.00 | 1.00 |
| | | Total (mg) | 81.00 | 81.00 | 81.00 | 81.00 |
| | | Mannitol | 187.30 | 187.30 | 187.30 | 187.30 |
| | | Corn starch | 52.76 | 52.76 | 52.76 | 52.76 |
| | | Crospovidone | 15.83 | 15.83 | 15.83 | 15.83 |
| | | Light anhydrous silicic acid | 2.64 | 2.64 | 2.64 | 2.64 |
| Disintegrative Particles | Granulation | Cellulose nanofibers (CNF-1) | - | - | - | - |
| | | Cellulose nanofibers (CNF-2) | - | - | - | - |
| | | Cellulose nanofibers (CNF-3) | - | - | - | - |
| | | Microfibrillated cellulose | - | - | 5.28 | - |
| | | Ethyl cellulose | 5.28 | - | - | 5.28 |
| | | Crystalline cellulose | - | 5.28 | - | - |
| | | Total (mg) | 263.80 | 263.80 | 263.80 | 263.80 |
| Sweetening agent | Mixing and tableting | Aspartame | 10.80 | 10.80 | 10.80 | 10.80 |
| Fluidizing agent | | Light anhydrous silicic acid | 0.90 | 0.90 | 0.90 | 0.90 |
| Lubricant | | Magnesium stearate | 3.6 | 3.6 | 3.6 | 3.6 |
| | Total (mg) | | 360 | 360 | 360 | 360 |
| | Tablet diameter (mm) | | 10 | 10 | 10 | 10 |

### [Example 2-1]

Particles and tablets were produced as in Example 1-1, except that the content of CNF was changed from 5.28 mg (2% by mass) to 2.64 mg (1% by mass). The tablets thus obtained were measured for compactibility, disintegrability, tablet hardness after humidification, and friability after humidification.

### [Example 3-1]

Particles and tablets were produced in Example 1-1, except that the content of CNF was changed from 5.28 mg (2% by mass) to 10.56 mg (4% by mass). The tablets thus obtained were measured for compactibility, disintegrability, tablet hardness after humidification, and friability after humidification.

### [Example 4-1]

Tablets were produced as in Example 1-1, except that the type of CNF was changed from "CNF-1" to "CNF-2". The tablets thus obtained were measured for compactibility, disintegrability, tablet hardness after humidification, friability after humidification, and tableting defects.

### [Example 5-1]

Tablets were produced as in Example 1-1, except that the type of CNF was changed from "CNF-1" to "CNF-3". The tablets thus obtained were measured for disintegrability, tablet hardness after humidification, friability after humidification, and tableting defects.

### [Example 6-1]

Tablets were produced as in Example 1-1, except that the punch was changed from "with marking" to "without marking" when tableting was performed by a rotary tableting machine. The tablets thus obtained were measured for compactibility and disintegrability.

### [Example 7-1]

Purified water was sprayed onto a mixed powder which included olmesartan medoxomil, hydroxypropyl cellulose (HPC-M, manufactured by Nippon Soda Co., Ltd.), and lactose hydrate (Fine Powder, manufactured by DFE Pharma K.K.). Then, a resultant mixture was granulated with use of a high-speed stirring mixer. Granules thus obtained were dried with use of a fluidized bed granulation device (MP-01, manufactured by Powrex Corp.) and then classified with use of sieves having respective mesh sizes of 250 µm and 75 µm. As a result, drug particles were prepared.

Next, 71 parts by mass of D-mannitol (PEARLITOL 50C, manufactured by Roquette Japan K.K.) and 1 part by mass of light anhydrous silicic acid (Adsolider-101, manufactured by Freund Corp.) were fed into a fluidized bed granulation device. CNF-1 was diluted by adding water so as to have a concentration of 1.25% (w/v), and then treated with a homogenizer at 10,000 rpm for 20 minutes (2 parts by mass as CNF). A resultant liquid was sprayed and granulation and then drying were carried out. Onto granules thus obtained, a liquid obtained by dispersing, in water, 20 parts by mass of corn starch (Corn Starch W, manufactured by Nihon Shokuhin Kako Co., Ltd.) and 6 parts by mass of crospovidone (polyplasdone INF-10, manufactured by ISP Japan Ltd.) was sprayed. Subsequently, granulation and then drying and sizing at 30 M were performed. As a result, disintegrative particles were obtained.

Then, drug particles, disintegrative particles, aspartame (manufactured by Ajinomoto Co., Inc.), light anhydrous silicic acid (Adsolider-101, manufactured by Freund Corp.), and magnesium stearate (vegetable, manufactured by Taihei Chemical Industrial Co., Ltd.) were mixed, and then tableted by a rotary tableting machine to a practical hardness. As a result, tablets with a diameter of 10 mm (punch: without marking, with score line) were obtained. The tablets thus obtained were measured for compactibility and disintegrability.

### [Example 8-1]

Cilostazol and D-mannitol (PEARLITOL 50C, manufactured by Roquette Japan K.K.) were charged into a fluidized bed granulation device. CNF-1 was diluted by adding water so as to have a concentration of 1.25% (w/v), and then treated with a homogenizer at 10,000 rpm for 20 minutes (2 parts by mass as CNF). In this liquid, corn starch (Corn Starch W, manufactured by Nihon Shokuhin Kako Co., Ltd.) and crospovidone (polyplasdone INF-10, manufactured by ISP Japan Ltd.) were dispersed in water. A liquid thus obtained was sprayed, and granulation and then drying and sizing at 30 M were performed. As a result, drug particles were obtained.

Thereafter, drug particles, crystalline cellulose (PH101, manufactured by Asahi Kasei Corp.), talc (Micro Ace P-3, manufactured by Nippon Talc Co., Ltd.), aspartame (manufactured by Ajinomoto Co., Inc.), light anhydrous silicic acid (Adsolider-101, manufactured by Freund Corp.), and magnesium stearate (vegetable, manufactured by Taihei Chemical Industrial Co., Ltd.) were mixed, and then tableted by a rotary tableting machine to a practical hardness. As a result, tablets with a diameter of 7 mm (punch: with marking, with score line) were obtained. The tablets thus obtained were measured for compactibility and disintegrability.

Table 6 shows composition of each component.

**[Table 6]**

| | Step | General Name | Example 8-1 | Comparative Example 5-1 |
|---|---|---|---|---|
| Drug particles | Granulation | Cilostazol | 50.00 | 50.00 |
| | | D-mannitol | 18.80 | 15.00 |
| | | D-mannitol | 15.00 | 15.00 |
| | | Corn starch | 27.50 | 27.50 |
| | | Crospovidone | 3.50 | 3.50 |
| | | Cellulose nanofibers (CNF-1) | 1.16 | - |
| | | Ethyl cellulose | - | 5.00 |
| | | Total (mg) | 115.96 | 116.00 |
| Excipient | Mixing and tableting | Crystalline cellulose | 10.00 | 10.00 |
| Lubricant | | Talc | 5.00 | 5.00 |
| Sweetening agent | | Aspartame | 2.00 | 2.00 |
| Fluidizing agent | | Light anhydrous silicic acid | 0.500 | 0.500 |
| Lubricant | | Magnesium stearate | 1.500 | 1.500 |
| | Total (mg) | | 135 | 135 |
| | Tablet diameter (mm) | | 7 | 7 |

### [Comparative Example 1-1]

Purified water was sprayed onto a mixed powder which included olmesartan medoxomil, hydroxypropyl cellulose (HPC-M, manufactured by Nippon Soda Co., Ltd.), and lactose hydrate (Fine Powder, manufactured by DFE Pharma K.K.). Then, a resultant mixture was granulated with use of a high-speed stirring mixer. Granules thus obtained were dried with use of a fluidized bed granulation device (MP-01, manufactured by Powrex Corp.) and then classified with use of sieves having respective mesh sizes of 250 µm and 75 µm. As a result, drug particles were prepared.

Next, 71 parts by mass of D-mannitol (PEARLITOL 50C, manufactured by Roquette Japan K.K.), 2 parts by mass of ethyl cellulose (ETHOCEL Standard FP 7CPS Premium, manufactured by DuPont Nutrition & Biosciences), and 1 part by mass of light anhydrous silicic acid (Adsolider-101, manufactured by Freund Corp.) were fed into a fluidized bed granulation device. In purified water, 20 parts by mass of corn starch (Corn Starch W, manufactured by Nihon Shokuhin Kako Co., Ltd.) and 6 parts by mass of crospovidone (polyplasdone INF-10, manufactured by ISP Japan Ltd.) were dispersed in water. A liquid thus obtained was sprayed, and granulation and then drying and sizing at 30 M were performed. As a result, disintegrative particles were obtained.

Thereafter, the drug particles, the disintegrative particles, aspartame (manufactured by Ajinomoto Co., Inc.), light anhydrous silicic acid (Adsolider-101, manufactured by Freund Corp.), and magnesium stearate (vegetable, manufactured by Taihei Chemical Industrial Co., Ltd.) were mixed, and then tableted by a rotary tableting machine to a practical hardness. As a result, tablets with a diameter of 10 mm (punch: with marking, with score line) were obtained. The tablets thus obtained were measured for compactibility, disintegrability, tablet hardness after humidification, friability after humidification, and tableting defects.

### [Comparative Example 2-1]

Tablets were produced as in Example 1-1, except that the type of CNF was changed from "CNF-1" to "crystalline cellulose". The tablets thus obtained were measured for particle size distribution, compactibility, disintegrability, tablet hardness after humidification, friability after humidification, and tableting defects.

### [Comparative Example 3-1]

Tablets were produced as in Example 1-1, except that the type of CNF was changed from "CNF-1" to "microfibrillated cellulose". The tablets thus obtained were measured for particle size distribution, compactibility, disintegrability, tablet hardness after humidification, friability after humidification, and tableting defects.

### [Comparative Example 4-1]

Tablets were produced as in Comparative Example 1, except that the punch was changed from "with marking" to "without marking" when tableting was performed by a rotary tableting machine. The tablets thus obtained were measured for compactibility and disintegrability.

### [Comparative Example 5-1]

Cilostazol, D-mannitol (PEARLITOL 50C, manufactured by Roquette Japan K.K.), and ethyl cellulose (ETHOCEL Standard FP 7CPS Premium, manufactured by DuPont Nutrition & Biosciences) were fed into a fluidized bed granulation device. In purified water, corn starch (Corn Starch W, manufactured by Nihon Shokuhin Kako Co., Ltd.) and crospovidone (polyplasdone INF-10, manufactured by ISP Japan Ltd.) were dispersed in water. A liquid thus obtained was sprayed, and granulation and then drying and sizing at 30 M were performed. As a result, drug particles were obtained.

Thereafter, the drug particles, crystalline cellulose (PH101, manufactured by Asahi Kasei Corp.), talc (Micro Ace P-3, manufactured by Nippon Talc Co., Ltd.), aspartame (manufactured by Ajinomoto Co., Inc.), light anhydrous silicic acid (Adsolider-101, manufactured by Freund Corp.), and magnesium stearate (vegetable, manufactured by Taihei Chemical Industrial Co., Ltd.) were mixed, and then tableted by a rotary tableting machine to a practical hardness. As a result, tablets with a diameter of 7 mm (punch: with marking, with score line) were obtained. The tablets thus obtained were measured for compactibility, disintegrability, and tableting defects.

### [Example 9-1]

### (Preparation of drug particles)

A CNF aqueous dispersion (CNF-1 described in <CNF used in Examples>) was sprayed onto a mixed powder which included levofloxacin hydrate (0.5 hydrate), hydroxypropyl cellulose (HPC-L FP (75 µm to 106 pm), manufactured by Nippon Soda Co., Ltd.), and low-substituted hydroxypropyl cellulose (LH-31, manufactured by Shin-Etsu Chemical Co., Ltd.), and granulation was carried out with use of a high-speed stirring mixer. Granules thus obtained were dried with use of a tumbling fluidized bed granulation device, and then classified with use of sieves having respective mesh sizes of 250 µm and 75 µm. As a result, drug particles were prepared.

Next, particle strength was measured with use of the drug particles obtained.

Table 7 shows composition of each component.

**[Table 7]**

| Step | General Name | Comparative Example 6-1 | Example 9-1 |
|---|---|---|---|
| | Levofloxacin hydrate | 25.620 | 25.620 |
| | Hydroxypropyl cellulose | 3.014 | 3.014 |
| Granulation | Low-substituted hydroxypropyl cellulose | 1.507 | 1.507 |
| | Cellulose nanofibers (CNF-1) | - | 0.084 |
| | Total (mg) | 30.141 | 30.225 |

### [Comparative Example 6-1]

Drug particles were prepared as in Example 9-1, except that water was used instead of the CNF aqueous dispersion.

Next, particle strength was measured with use of the drug particles obtained.

### [Example 10-1]

### (Preparation of drug particles)

A CNF aqueous dispersion (CNF-1 described in <CNF used in Examples>) was added to a mixed powder which included levofloxacin hydrate (0.5 hydrate), hydroxypropyl cellulose (HPC-L FP, manufactured by Nippon Soda Co., Ltd.), crystalline cellulose (Ceolus PH101, manufactured by Asahi Kasei Corp.), and carmellose (NS-300, manufactured by Nichirin Chemical Industries, Ltd.), and granulation was carried out with use of a high-speed stirring mixer. Granules thus obtained were dried with use of a tumbling fluidized bed granulation device, and then classified with use of a sieve having a screen diameter of 1143 mm. As a result, drug particles were sized.

Thereafter, the drug particles, croscarmellose sodium (Ac-Di-Sol, manufactured by DuPont), and magnesium stearate (vegetable, manufactured by Taihei Chemical Industrial Co., Ltd.) were mixed, and then tableted by a rotary tableting machine using an oval punch (having a major axis of 8.1 mm and a minor axis of 4.7 mm) to a practical hardness. Thus, tablets were obtained.

Table 8 shows composition of each component.

**[Table 8]**

| | Step | General Name | Example 10-1 | Comparative Example 7-1 |
|---|---|---|---|---|
| Drug particles | Granulation | Levofloxacin hydrate | 256.20 | 256.20 |
| | | Crystalline cellulose | 20.85 | 23.05 |
| | | Carmellose | 26.50 | 26.50 |
| | | Hydroxypropyl cellulose | 6.25 | 6.25 |
| | | Cellulose nanofibers (CNF-1) | 2.20 | - |
| | | Total (mg) | 312.00 | 312.00 |
| Disintegrator | Mixing and tableting | Croscarmellose sodium | 10.00 | 10.00 |
| Lubricant | | Magnesium stearate | 1.600 | 1.600 |
| Total (mg) | | | 324 | 324 |

### [Comparative Example 7-1]

Drug particles were prepared as in Example 10-1, except that water was used instead of the CNF aqueous dispersion. Next, tablets were obtained as in Example 10-1 with use of the drug particles obtained. The tablets obtained were measured for compactibility, disintegrability, and tableting defects.

### [Results]

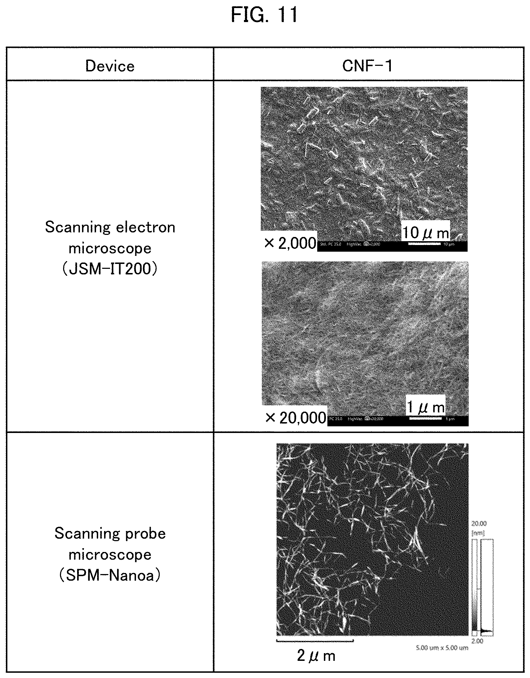

Figs. 11 to 18 show results. As shown in Fig. 11, CNF-1 that was used in Examples was fine. Although an attempt was made to measure the average fiber length of CNF-1 with use of Valmet FS5 (having a detection sensitivity of 10 µm or more), which is a successor to FS-200 disclosed in Japanese Patent Application Publication Tokukai No. 2009-203559, it was not possible to measure the average fiber length since the average fiber length was below a detection limit of Valmet FS5. Further, on the basis of image data shown in Fig. 11, the average fiber length of CNF-1 was estimated to be less than 10 µm.

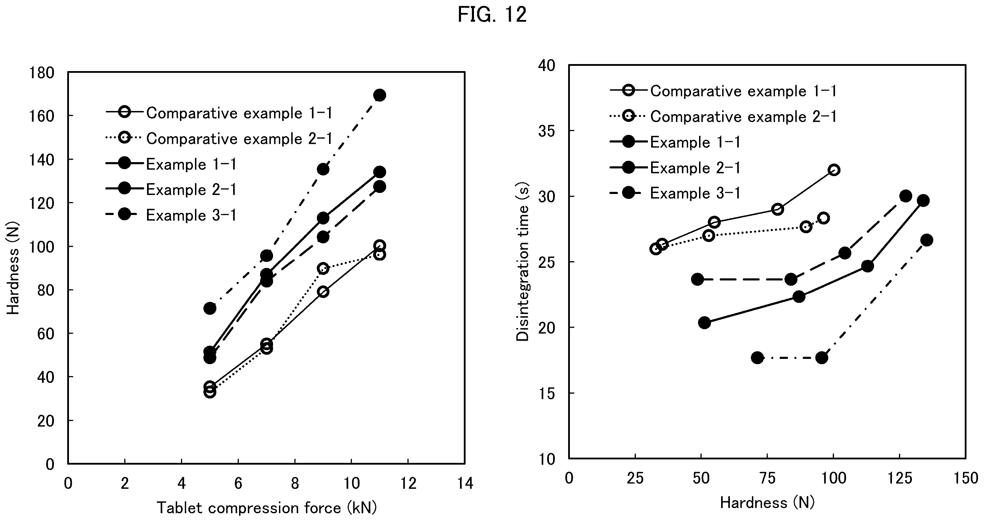

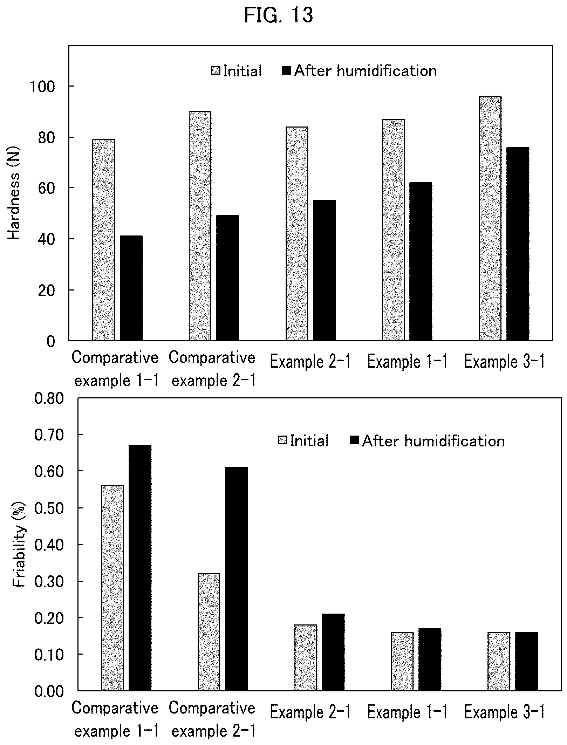

It was clear from Figs. 12 and 13 that Examples had better compactibility and better disintegrability than Comparative Examples. Further, Examples had better tablet hardness after humidification and better friability after humidification than Comparative Examples. In addition, among Examples, Example 3-1 having a large CNF content had the shortest disintegration time.

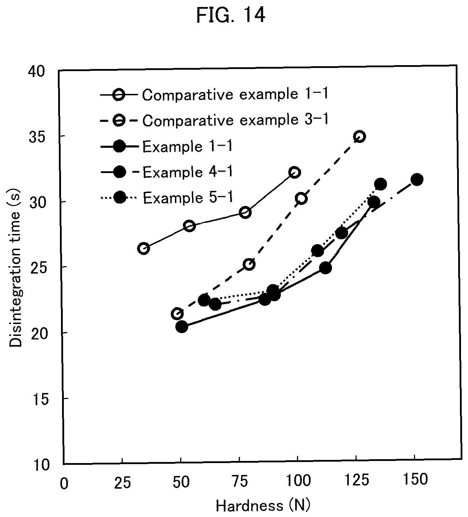

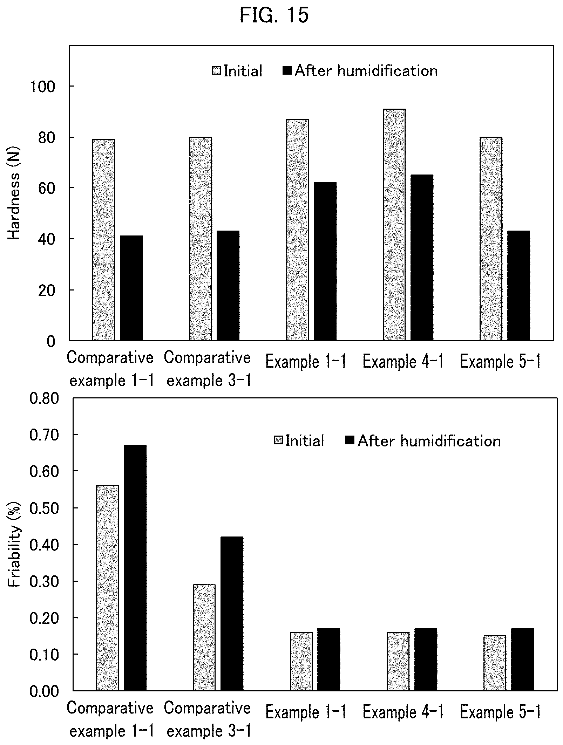

It was clear from Figs. 14 and 15 that CNF which was contained in tablets and which had a shorter average fiber length (in particular, in a case where the average fiber length was less than 10 µm), the disintegrability, the tablet hardness after humidification, and the friability after humidification were better.

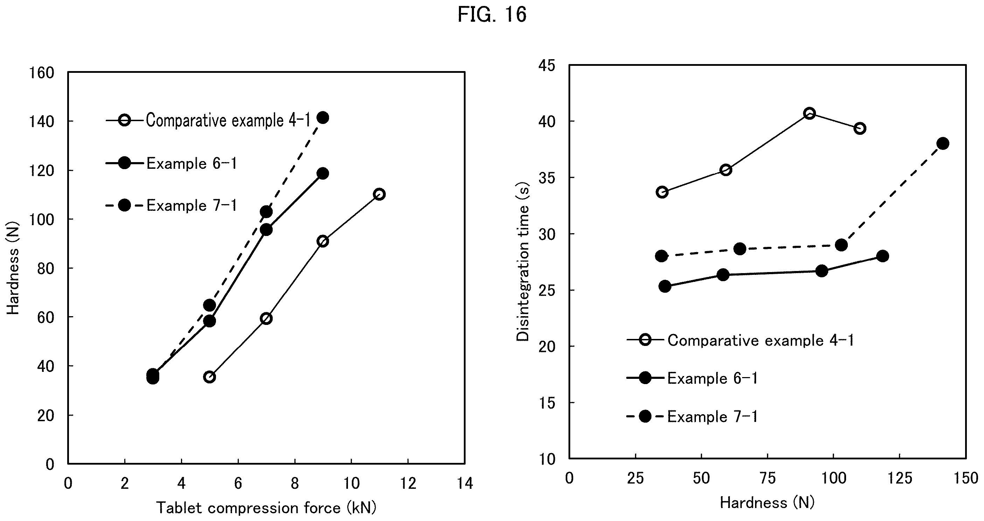

It was clear from Fig. 16 that Examples 6-1 and 7-1 had better compactibility and better disintegrability than Comparative Example 4-1. In addition, among Examples, Example 6-1 having a large ratio of CNF present at particle surfaces had the shortest disintegration time.

Table 9 shows a result of evaluating tableting defects. In Examples 1-1, 4-1, 5-1, and 8-1, no sticking (adhesion of powder) occurred, whereas in Comparative Examples 1-1, 2-1, 3-1, and 5-1, sticking occurred.

**[Table 9]**

| | Example 1-1 | Example 4-1 | Example 5-1 | Comparative Example 1-1 | Comparative Example 2-1 | Comparative Example 3-1 |
|---|---|---|---|---|---|---|
| Tableting defects | A | A | A | B | B | B |

| | Example 8-1 | Comparative Example 5-1 |
|---|---|---|
| Tableting defects | A | C |

It was shown from the above that in the present orally disintegrating tablet, at least one of compactibility, disintegrability, tableting defects, tablet hardness after humidification, and friability after humidification was improved.

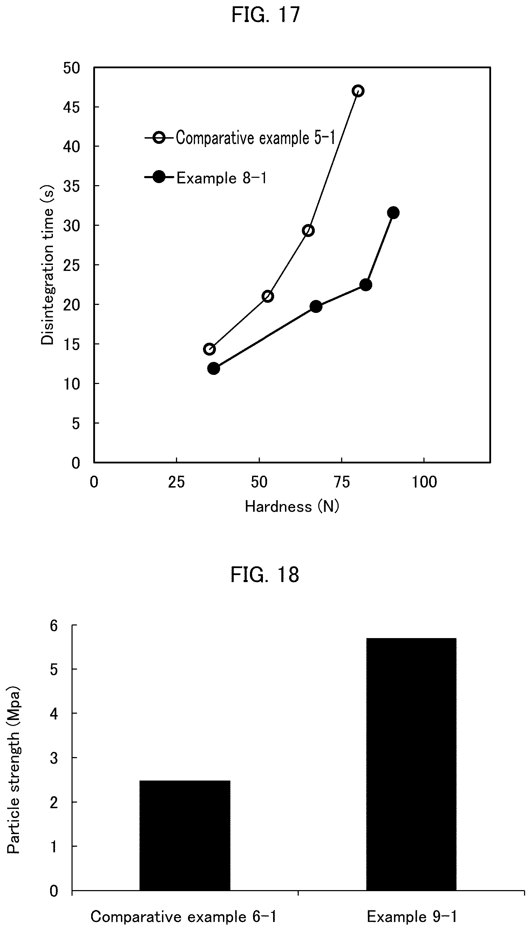

It was clear from Fig. 17 that, also in a case where drug particles contain CNF, the examples had better disintegrability than the comparative examples.

It is clear from Fig. 18 that the particle strength is higher in Example 9-1 than in Comparative Example 6-1. Therefore, it was shown that the particles according to an embodiment of the present invention, which contained a drug and CNF having an average particle diameter of less than 10 µm, had improved particle strength.

Furthermore, it was found from Table 10 below that Example 10-1 had better disintegrability and better suppression of tableting defects than Comparative Example 7-1 while having a hardness similar to that of Comparative Example 7-1. Therefore, it has been found that tablets that include particles according to an embodiment of the present invention, which contain a drug and CNF having an average particle diameter of less than 10 µm, have excellent compactibility, excellent disintegrability, and excellent suppression of tableting defects.

**[Table 10]**

| | Example 10-1 | Comparative Example 7-1 |
|---|---|---|
| Hardness | 73 N | 79 N |
| Disintegration time | 3 min 30 s | 4 min |
| Tableting defects | A | B |

### Industrial Applicability

### (Invention 1)

Since the present particle has ensured strength, the present particle is suitably used in various fields in which particles are used, for example, the fields of pharmaceuticals, foods, cosmetics, and the like.

### (Invention 2)

Since the present orally disintegrating tablet has improved various physical properties, the present orally disintegrating tablet is suitably used in fields in which an orally disintegrating tablet is used, for example, the fields of pharmaceuticals and foods.

### Reference Signs List

1 core particle
2 coating layer
3 intermediate layer

## Claims

1. A particle, wherein:
a core particle is coated with a coating layer; and
the coating layer contains cellulose nanofibers.

2. The particle according to claim 1, further comprising an intermediate layer between the core particle and the coating layer.

3. The particle according to claim 1, wherein the coating layer contains no additive other than the cellulose nanofibers.

4. The particle according to claim 1, wherein the cellulose nanofibers have an average fiber diameter of less than 1 µm.

5. A composition comprising particles recited in any one of claims 1 to 4.

6. A composition according to claim 5, wherein the composition is applied to at least one selected from the group consisting of pharmaceuticals, foods, and cosmetics.

7. A method for producing a particle in which a core particle is coated with a coating layer, the method comprising the step of
coating the core particle with a coating layer containing cellulose nanofibers.

8. A method for ensuring strength of a particle in which a core particle is coated with a coating layer, the method comprising the step of
causing the coating layer to contain cellulose nanofibers.

9. An orally disintegrating tablet comprising particles that contain cellulose nanofibers having an average particle diameter of less than 10 µm.

10. The orally disintegrating tablet according to claim 9, wherein the particles are disintegrative particles.

11. The orally disintegrating tablet according to claim 9 or 10, wherein the cellulose nanofibers have an average fiber diameter of 0.001 µm to 1 µm.

12. The orally disintegrating tablet according to claim 9 or 10, wherein:
the particles have a core and a coating layer; and
the cellulose nanofibers are contained in the coating layer.

13. The orally disintegrating tablet according to claim 10, wherein:
the disintegrative particles include a sugar alcohol, and organic and inorganic, hydrophilic and water-insoluble additives;
the organic hydrophilic and water-insoluble additive includes at least one selected from the group consisting of starch, a starch derivative, and crospovidone; and
the inorganic hydrophilic and water-insoluble additive includes light anhydrous silicic acid, magnesium aluminometasilicate, or a combination thereof.

14. An orally disintegrating tablet comprising cellulose nanofibers having an average fiber diameter of less than 10 µm.

15. A disintegrative particle comprising cellulose nanofibers having an average particle diameter of less than 10 µm.

16. A particle comprising a drug and cellulose nanofibers having an average particle diameter of less than 10 µm.

17. A method for producing an orally disintegrating tablet including particles, the method comprising the step of
causing the particles to contain cellulose nanofibers having an average particle diameter of less than 10 µm.

18. A method for improving physical properties of an orally disintegrating tablet including particles, the method comprising the step of
causing the particles to contain cellulose nanofibers having an average particle diameter of less than 10 µm,
the physical properties being at least one selected from the group consisting of the following physical properties:
(1) compactibility;
(2) disintegrability;
(3) tableting defects;
(4) tablet hardness after humidification; and
(5) friability after humidification.
